# EUROPEAN PATENT APPLICATION

(11) **EP 3 907 288 A1**
(43) Date of publication of application: **10.11.2021**
(21) Application number: 21165156.7
(22) Date of filing: 30.04.2015
(51) Int. Cl.: C12N 15/113, C12N 15/117, A61K 31/7088, A61P 29/00

(54) **PLANT SRNA EXTRACT FOR USE AS AN IMMUNOSUPPRESSIVE AGENT**

(30) Priority: 30.04.2014 WO PCT/EP2014/058888
(62) Divisional of application: 15720695.4
(71) Applicant: Fondazione Edmund Mach, 38010 San Michele All'adige (TN) (IT)
(72) Inventor: SI AMMOUR, Azeddine, 38121 Trento (IT); VIOLA, Roberto, 38010 Località San Donà (IT); CAVALIERI, Duccio, 50019 Sesto Fiorentino,Firenze (IT); RIZZETTO, Lisa, 38030 Giovo (IT)
(74) Representative: Turri, Elisa

(57) **Abstract**

The present invention relates to a plant sRNA extract for use as an immunosuppressive agent.

## Description

The present invention relates to a plant sRNA extract for use as an immunosuppressive agent.

The present invention further relates to a composition comprising 2 or more plant sRNA extracts for use as an immunosuppressive agent.

The present invention relates to a plant sRNA extract for use as an anti-inflammatory agent.

The present invention further relates to a composition comprising 2 or more plant sRNA extracts for use as an anti-inflammatory agent.

A wide variety of eukaryotic organisms, including plants, animals, and fungi, have evolved several RNA-silencing pathways to protect their cells and genomes against invading nucleic acids, such as viruses or transposons, and to regulate gene expression during development or in response to external stimuli (for review, see Baulcombe (2005) Trends Biochem. Sci. 30: 290-293; Meins et al. (2005) Annu. Rev. Cell Dev. Biol. 21:297-318).

Non-coding RNAs (ncRNAs) are functional RNA molecules that are transcribed from DNA but are not translated into proteins. In general. ncRNAs function to regulate gene expression at the transcriptional and post-transcriptional level. The short ncRNAs (<30 nt) encompass microRNAs (miRNAs), short interfering RNAs (siRNAs), and piwi-interacting RNAs (piRNAs) (Sharma et al. (2013), Mol Biotechnol 55, 63:77). MicroRNAs are known to interfere with gene expression in a sequence-specific manner in most organisms (although differently in plants and animals). This regulation has been mainly investigated studying miRNA acting within a given cell type through binding to regulatory regions on the nuclear DNA (Gomes AQ et al (2013) Int. J. Mol. Sci, 14, 16010-16039).

Over the past decade a substantial amount of scientific literature has highlighted the role played by sRNA species, and particularly miRNA, in the immune functions (for a review see Bi Y. et al.; MicroRNAs: novel regulators during the immune response. J Cell Physiol. 2009 Mar;218(3):467-72; O'Connell R.M. et al.; Physiological and pathological roles for microRNAs in the immune system. Nat Rev Immunol. 2010 Feb;10(2):111-22). Endogenous miRNAs have been proposed as therapeutics or targets in the cure of cancer and other disorders (Calin G.A and Croce CM; MicroRNA signatures in human cancers. Nat Rev Cancer. 2006 Nov;6(11):857-66). The main limiting step to this therapeutic application is that canonical siRNA duplexes are potent activators of the mammalian innate immune system (Robbins et al., siRNA and innate immunity, Oligonucleotides 2009; 19(2):89-102) and therefore show undesired off-effects such as their induction of pro-inflammatory processes (Judge AD et al; Sequence-dependent stimulation of the mammalian innate immune response by synthetic siRNA. Nat Biotechnol 2005; 23(4): 457-62; Sioud, Induction of inflammatory cytokines and interferon responses by double-stranded and single-straned siRNA is sequence-dependent and requires endosomal localization. J Mol Biol, 2005; 348(5):1079-90). This is not surprising *per se,* given the fact that a number of viruses are constituted by double stranded RNAs and during evolution the mammalian host has developed Toll Like Receptor (TLR) mediated responses to keep viruses under immune control (Kawai and Akira; Immune recognition of viral infection. Nature Immunology 2006; 7:131-137). In particular double stranded RNAs are recognized by TLR3 (Alexopolou et al; Recognition of double-stranded RNA and activation of NF-kappaB by Toll-like receptor 3. Nature 2001; 413: 732-738) and single stranded RNAs are recognized by TLR7 and TLR8 (Hornung et al; RNA recognition via TLR7 and TLR8. Handb Exp Pharmacol 2008; 183:71-86). Thus we can generalize that endogenous miRNAs act as modulators of immune responses by regulating gene expression, while exogenous small RNAs (sRNA) are recognized by immune cells as potentially harmful eliciting mainly inflammatory responses.

Recent studies have found sRNAs contained within "exosomes", i.e. extracellular vesicles composed of lipids and proteins, which are exchanged between cells and organs in mammals (for a review see van der Grein and Nolte-'t-Hoen ;"Small Talk" in the innate immune system via RNA-containing extracellular vesicles. Front Imm 2014; 5:542; Robbins and Morelli; Regulation of immune responses by extracellular vesicles. Nat Rev Immunol. 2014; 14(3): 195-208). Exosomes are produced by both immune and non-immune cells and play an important role in the regulation of immunity by mediating immune stimulation or suppression and by driving inflammatory, autoimmune and infectious disease pathology. Since many sRNAs can regulate gene expression, their intercellular transfer via exosome may cause long-term changes in the function of exosome-targeted cells.

The sRNA content with a length of <100 nucleotides of human immune cell-derived exosomes has been recently characterized by deep sequencing and found to consist of a large variety of small non-coding RNA species (Nolte-'t Hoen et al; Deep sequencing of RNA from immune cell-derived vesicles uncovers the selective incorporation of small non-coding RNA biotypes with potential regulatory functions. Nucleic Acids Res 2012, 40(18):9272-85) with possible gene regulatory functions (Kapranov et al. RNA maps reveal new RNA classes and a possible function for pervasive transcription. Science 2007, 316(5830):1484-8; Persson et al; The non-coding RNA oft he multidrug resistance-linked vault partcle encodes multiple regulatory small RNAs. Nat Cell Biol 2009, 11(10):1268-71. Haussecker et al; Human tRNA-derived small RNAs in the global regulation of RNA silencing. RNA 2010, 16(4):673-95).

Exosomes or other RNAs shuttle vesicles, not only constitute a mechanism for intra- and intercellular sRNAs transfer, but also offer protection from RNAse degradation (Valadi et al; Exosome-mediated transfer of mRNA and microRNA is a novel mechanism of genetic exchange between cells. Nat Cell Biol, 2007; 9:654:659).

In addition to the known effects of intracellular or intraspecific miRNAs, a more profound change of paradigm has been proposed by the appearance of evidences that indicate the potential of plant miRNAs to modulate human cells (Zhang L et al., Exogenous plant MIR168a specifically targets mammalian LDLRAP1: evidence of cross-kingdom regulation by microRNA. Cell Research, 2012 22:107-126). In a recent highly debated report, Zhang et al (supra) discovered that the specific rice MIR168a could specifically target the mammalian low-density lipoprotein receptor adapter protein, decreasing its expression in liver with consequence on LDL levels. If validated, diet-derived foreign microRNA absorption and function in consuming vertebrates would drastically alter our understanding of nutrition and ecology. Therapeutic exploitation of RNAi in treating human disease is difficult because these accessory processes are absent or diminished in most animals. A recent report challenged multiple paradigms, suggesting that ingested microRNAs (miRNAs) are transferred to blood, accumulate in tissues, and exert canonical regulation of endogenous transcripts (see review by Witwer and Hirsch (2014), Transfer and functional consequences of dietary microRNAs in vertebrates: Concepts in search of corroboration, BioAssays).

Recently it has been shown another exogenous and inter-organism exchange of ncRNAs. Nematode parasites use secretion of miRNA-containing exosomes to manipulate and modulate host innate response by direct target gene regulation (Buck et al; Exosomes secreted by nematode parasites transfer small RNA to mammalian cells and modulate innate immunity.Nat Comm 2014; 5:5488).

It has recently been hypothesized that exosomes endocytosis by target cells involves docking to extracellular matrix protein followed by fusion with the underlying cell cytoskeleton and release of content in the intracellular lumen (Tian et al; Dynamics of exosome internalization and trafficking. J Cell Physiol 2013; 228:1487-1495; Morelli et al; Endocytosis, intracellular sorting, and processing of exosomes by dendritic cells.Blood 2004; 104:3257-3266; Clayton et al; Adhesion and signaling by B cell-derived exosomes: the role of integrins. FASEB J 2004; 18:977-979).

Although it was initially thought that dsRNA of at least 30 bp is required to activate siRNA non-sequence-specific immune suppression through the type I IFN system (Elbashir et al; Duplexes of 21-nucleotides RNAs mediate RNA interference in cultured mammalian cells. Nature 2001; 411:494-8), it later emerged that also siRNA of less than 21 bp can induce inflammation through Toll-like receptor signalling (Tatematsu et al; Beyond dsRNA: Toll-like receptor 3 signalling in RNA-induced immune responses. Biochem J. 2014,1;458(2):195-201; Judge AD et al; Sequence-dependent stimulation of the mammalian innate immune response by synthetic siRNA. Nat Biotechnol 2005; 23(4): 457-62; Sioud, Induction of inflammatory cytokines and interferon responses by double-stranded and single-stranded siRNA is sequence-dependent and requires endosomal localization. J Mol Biol, 2005; 348(5):1079-90;Bridge et alInduction of an interferon response by RNAi vectors in mammalian cells. Nat Genet 2003; 34(3):263-4; Sledz et al; Activation of the interferon system by short-interfering RNAs. Nat Cell Biol 2003; 5:834).

The evidence that intercellular exchange of miRNAs via exosomes or other vesicles control inflammation-mediated processes including tumor development is increasing. Experiments on tumors induced in the animal model have shown exosome transported <19 bp miRNA binding to TLR7. In particular, it was shown that tumor-secreted miR-21 and miR-29a can function by non-canonical mechanism, by binding as ligands to receptors of the Toll-like receptor (TLR) family, murine TLR7 and human TLR8, in immune cells, triggering a TLR-mediated prometastatic inflammatory response that ultimately may lead to tumor growth and metastasis. (Fabbri et al; MicroRNAs bind to Toll-like receptors to induce prometastatic inflammatory response. PNAS 2012, 109(31): E2110-E2116).

This is the first evidence of direct binding to TLRs of human produced exosome-contained miRNAs.

Several pieces of evidence indicate a proinflammatory action of siRNA and sRNA. These molecules have been shown to signal in a sequence-independent manner through the TLR3 pathway, inducing cellular activation and type I IFN response with a mechanism similar to that of viral dsRNA (Karikò et al; Small interfering RNAs mediate sequence-independent gene suppression and induce immune activation by signaling through toll-like receptor 3. J Imm 2004; 172:6545-6549).

The finding that 2'-O-methyl-modified duplex RNA with a length of >36 bp competes with TLR3 binding and abolishes the IFN-β-inducing ability of PolyI:C (Okahira et al; Interferon-beta induction through toll-like receptor 3 depends on double stranded RNA structure. DNA and Cell Biol 2005; 24(10): 614-23) further indicates that sRNA interference with immune function occurs through non-canonical pathways.

There is always a need for agents and compositions which can be used as immunosuppressive agents, and which can be used for the prevention or treatment of inflammatory disorders.

Our experiments surprisingly revealed that exogenous administration of DOTAP vesicles containing plant sRNAs extract(s) exhibit an immunosuppressive and anti-inflammatory effect: plant sRNA extracts and plant miRNAs dampen inflammation *per se* and also prevent inflammation by inhibiting T cell proliferation during an inflammatory condition.

Moreover, we could surprisingly show that plant sRNA extracts interact with dendritic cells through TLR3 and negatively regulate the expression of TIR-domain containing adapter inducing IFNβ (TRIF) and IFNβ genes during inflammatory condition.

Thereby, we could surprisingly show that plant sRNA extracts interact with dendritic cells through TLR3, which represents a protein, and consequently the interaction takes place by a sequence-independent manner.

Accordingly, plant miRNA, plant sRNA extracts, and compositions comprising 2 or more plant sRNA extracts represent novel immunosuppressive agents, which may be used in particular for treating and preventing inflammatory diseases, in particular autoimmune diseases or inflammatory diseases such as multiple sclerosis, rheumatoid arthritis, immune mediated renal disease, hepatobiliary diseases, inflammatory bowel disease (IBD), psoriasis, and asthma.

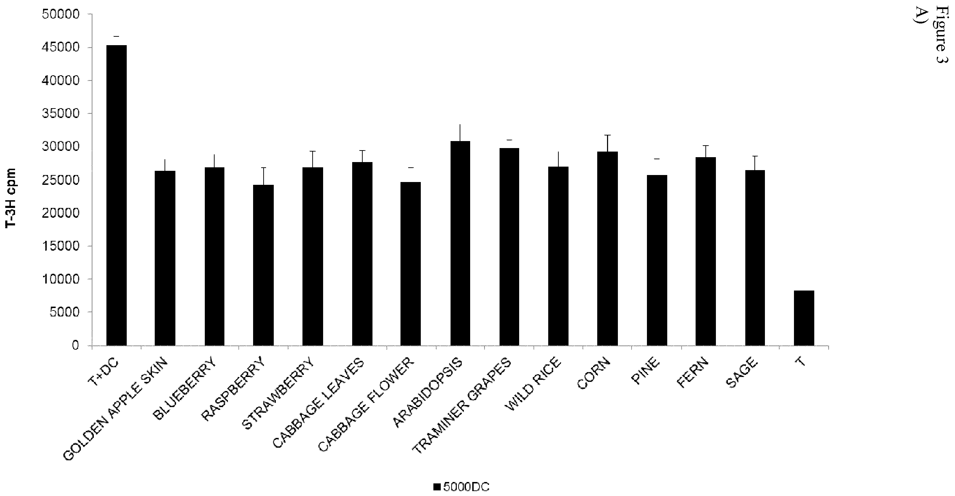

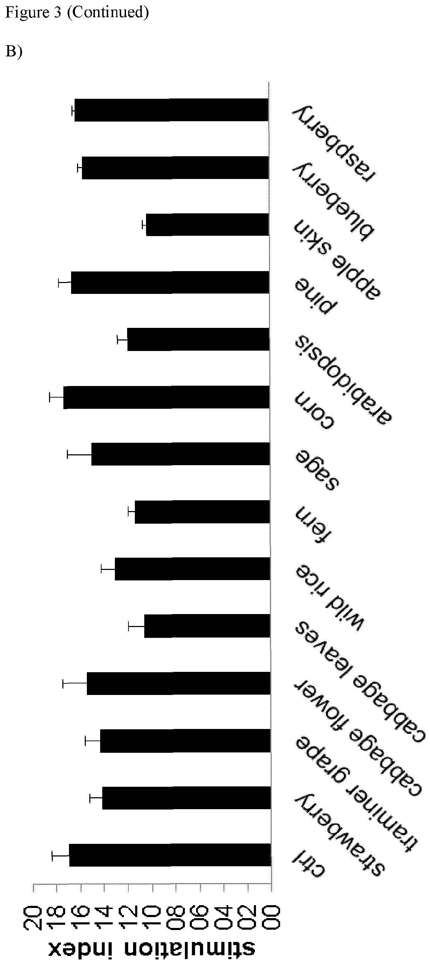

The in *vitro* test for assessing the function of T lymphocytes under the stimulation of specific inflammatory agents revealed that the presence of various plant sRNA extracts decreases T lymphocytes proliferation (see Figures 3A and 3B).

Phenotypic evaluation of DC and T lymphocytes additionally showed that the presence of plant sRNA extracts alters cells differentiation pathway.

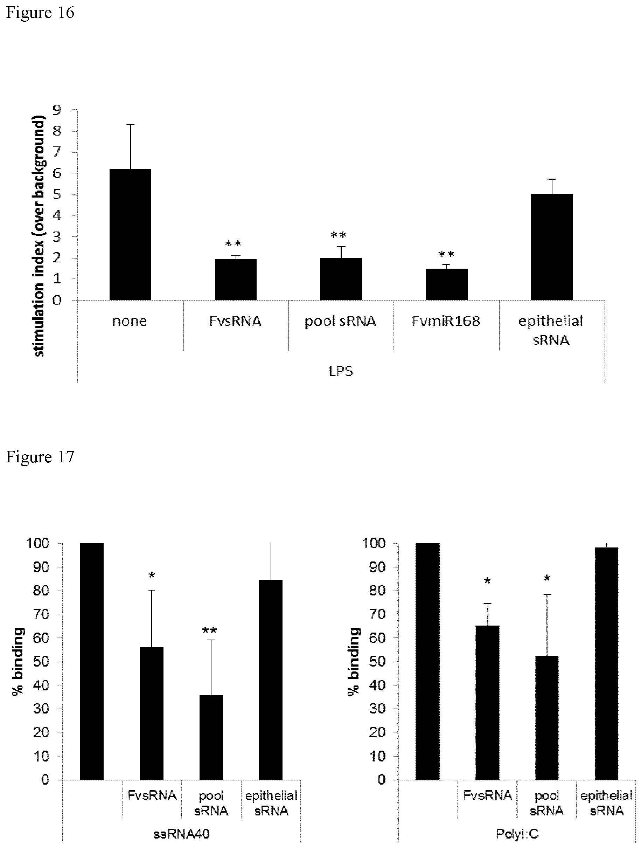

We were surprisingly further able to show that plant sRNA extracts encapsulated into liposomes were able to contact DCs and, by endocytosis, contact TLR3 and interfere with TRIF-mediated signaling (see figures 16 to 20).

Moreover, we surprisingly demonstrated that plant sRNA extracts exhibit an immunosuppressive and anti-inflammatory effect in a sequence-independent manner, whereas human sRNA extract does not exhibit such effect (see e.g. Figures 16 to 18).

Accordingly, sRNA extract obtained from animal meat did not exhibit an immunosuppressive and anti-inflammatory effect in the *in vitro* tests (data not shown), but rather has a potential pro-inflammatory effect, in agreement with existing information (Alexopolou et al; Recognition of double-straned RNA and activation of NF-kappaB by Toll-like receptor 3.Nature 2001; 413: 732-738, Hornung et al; RNA recognition via TLR7 and TLR8. Handb Exp Pharmacol 2008; 183:71-86, Fabbri et al; MicroRNAs bind to Toll-like receptors to induce prometastatic inflammatory response. PNAS 2012, 109(31): E2110-E2116).

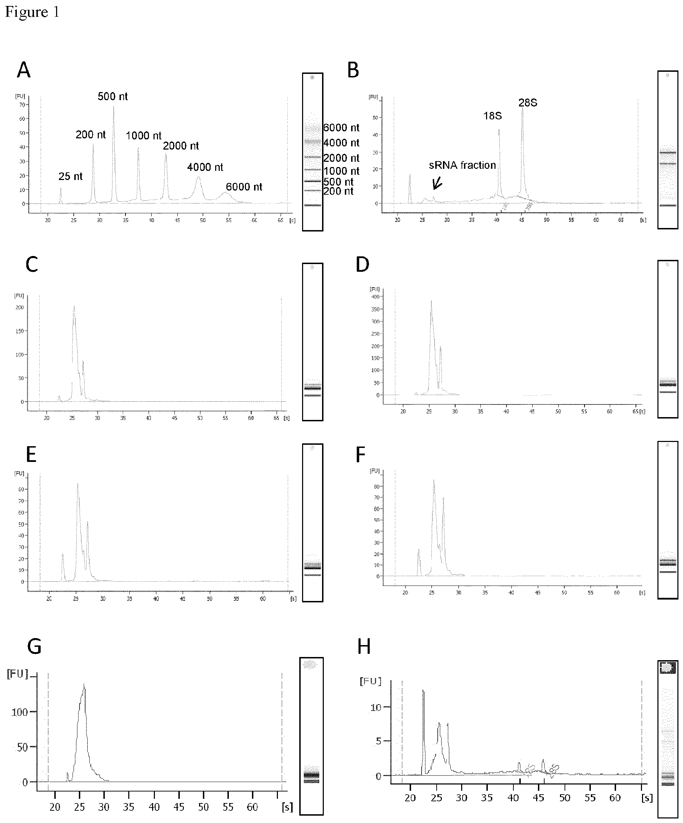

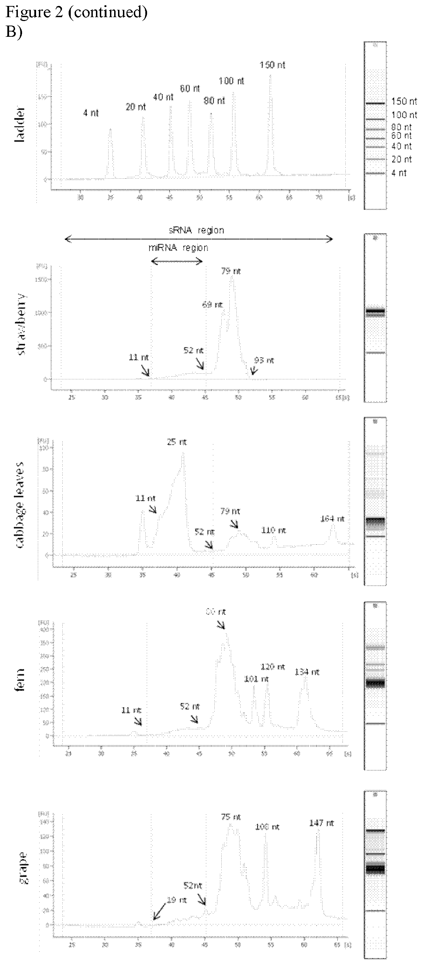

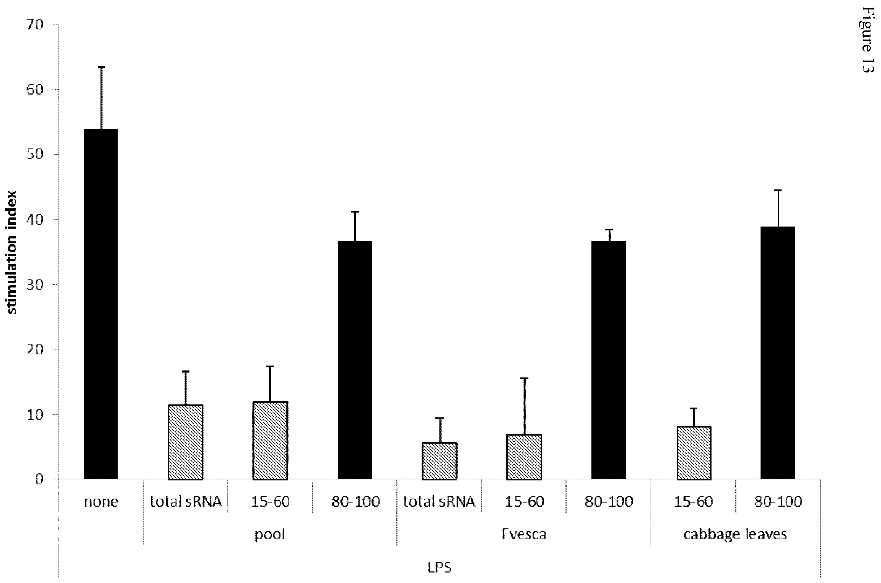

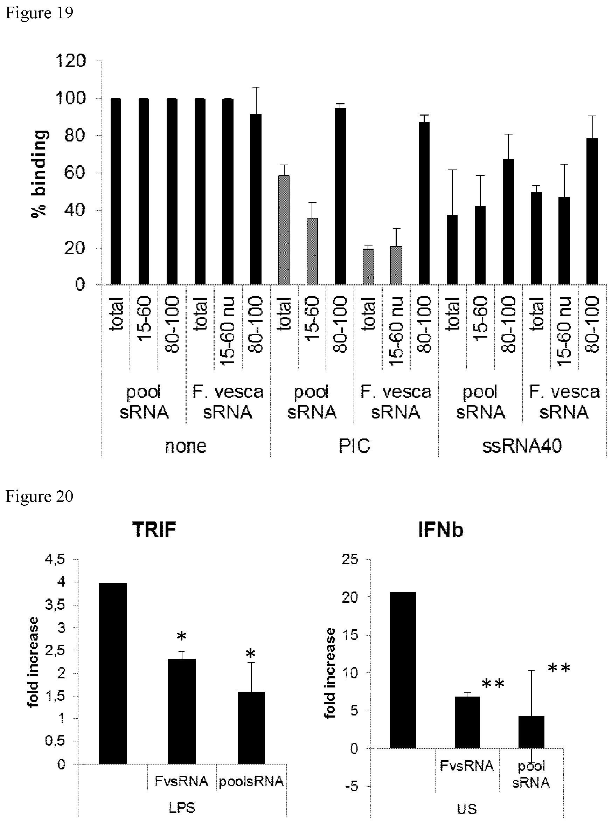

We were further able to show that various plant sRNA extracts, such as strawberry (*Fragaria vesca*) fruit plant sRNA extract (in the experiments: "Fv sRNA" or "FvsRNA") and compositions comprising plant sRNA extracts from 3 different, unrelated plant varieties (compositions comprising plant sRNA extracts from fern leaves, cabbage leaves, and apple skin; in the experiments: "pool") exhibit the immunosuppressive and anti-inflammatory effect (Figures 3A, 3B), in particular plant sRNA extracts comprising the sRNA of a plant sample having a length of 10 to 120 nucleotides (Example 1, Figures 1, 2B), in particular plant sRNA extracts comprising sRNA of a plant sample having a length of 10 to 60 or 15 to 60 nucleotides (Example 5; Figures 13, 19).

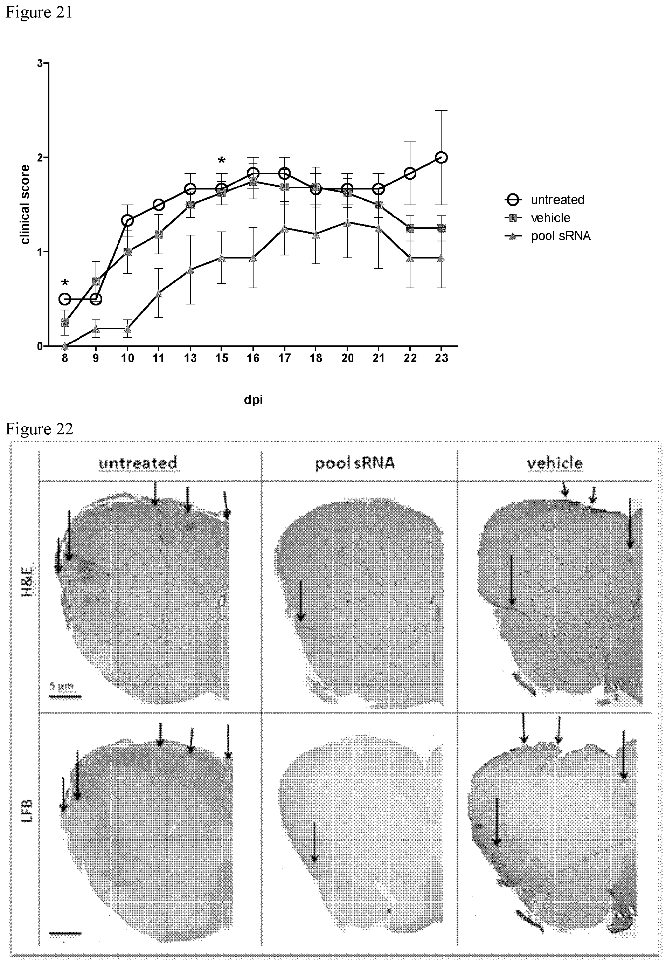

Moreover, the *in vivo* experiment on the murine model for Experimental Autoimmune Encephalopathy, an established animal model system for multiple sclerosis (Mix et al; Animal models of multiple sclerosis-potentials and limitations. Prog. Neurobiol 2010; 92: 386-404), also performed for purpose of this invention, surprisingly demonstrates that plant sRNA extracts and compositions comprising 2 or more plant sRNA extracts of the invention decrease inflammation during multiple sclerosis and ameliorate the pathological conditions (Figures 21 to 24).

Therefore, it could surprisingly be shown that plant sRNAs and plant sRNA extracts, in contrast to human sRNAs, human miRNAs and double-stranded viral RNAs reduce inflammation, instead of eliciting inflammatory signals. This mechanism is mediated by plant sRNAs binding and saturating Toll like Receptor 3 (TLR3) and affecting downstream signaling via TRIF in a way that reduces inflammatory signals sensed also by other TLRs. Therefore, the antinflammatory effects exhibited by plant sRNA extracts in animals, in particular humans, are mediated by binding to protein targets rather than by direct regulation of gene expression through binding to specific sRNA sequences (i.e. they exhibit a sequence-independent bioactivity).

Further, we could surprisingly show that the plant sRNA extracts can be administered and delivered efficiently to an animal to be treated by a formulation comprising liposomes (see Figures 21 and 22; formulation: plant sRNA extracts dissolved in phosphate buffered saline solution and generation of liposomes with DOTAP, obtainable by incubation of plant sRNA extracts with DOTAP for 30 minutes at 37°C).

In plants, RNA-silencing pathways have been shown to control a variety of developmental processes including flowering time, leaf morphology, organ polarity, floral morphology, and root development (reviewed by Mallory and Vaucheret (2006), Nat, Genet, 38:S31-36). All RNA-silencing systems involve the processing of double-stranded RNA (dsRNA) into small RNAs of 21 to 25 nucleotides (nt) by an RNaseIII-like enzyme, known as Dicer or Dicer-like in plants (Bernstein et al. (2001) Nature 409: 363-366; Xie et al. (2004) PLoS Biol 2, E104:0642-0652; Xie et al. (2005) Proc Natl Acad Sci USA 102: 12984-12989; Dunoyer et al., (2005) Nat Genet 37: 1356-1360). These small RNAs are incorporated into silencing effector complexes containing an Argonaute protein (for review, see Meister and Tuschl (2004) Nature 431: 343-349).

There are a number of major differences between plant and animal miRNA: Plant microRNA precursors are transcribed by RNA polymerase II as in animals. However, in contrast to animals the entire process of plant microRNA biogenesis is undertaken within the plant nucleus. The mature microRNAs are exported out of the nucleus by Hasty, an exportin 5-like protein found in plants. There are fundamental differences between plant and animal microRNAs (Axtell, MJ., et al. Vive la difference: biogenesis and evolution of microRNAs in plants and animals. Genome Biology. 12(2011): p. 221-234.):
a) In animals miRNA precursors are sequentially processed by Drosha followed by Dicer to produce miRNA duplexes whereas only one Dicer-like enzyme (DCL1) is required in plants.
b) In plants miRNA duplexes are methylated at the 2'OH group at the 3' termini.
c) The target recognition process between plants and animals is different. In plants miRNAs direct mRNA cleavage of a microRNA target whereas in animals miRNAs usually inhibit translation of their targets.
d) In contrast to the nearly perfect match of plant miRNAs to the exons of their target, hybridization of microRNA to targets in animals is less stringent and only a canonical 7-8 nucleotide "seed sequence" is known to be specific for microRNA target recognition.

Studies of plant miRNA focus on the role of plant miRNAs in plant; e.g. by the development of artificial miRNAs. Artificial microRNAs (amiRNAs) have been described in *Arabidopsis* targeting viral mRNA sequences (Niu et al. (2006) Nature Biotechnology 24: 1420-1428) or endogenous genes (Schwab et al. (2006) Plant Cell 18: 1121-1133). The amiRNA construct can be expressed under different promoters in order to change the spatial pattern of silencing (Schwab et al. (2006) Plant Cell 18: 1121-1133). Artificial miRNAs replace the microRNA and its complementary star sequence in a miRNA precursor backbone and substitute sequences that target an mRNA to be silenced.

Silencing by endogenous miRNAs can be found in a variety of spatial, temporal, and developmental expression patterns (Parizotto et al. (2007) Genes Dev 18:2237-2242; Alvarez et al. (2006) Plant Cell 18: 1134-51).

As specific plant miRNA molecules are known to target plant target sequences, the effect of plant miRNAs in animals is unknown.

Immune-related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multi-step pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

Many immune related diseases are known and have been extensively studied. Such diseases include immune-mediated inflammatory diseases such as rheumatoid arthritis, immune mediated renal disease, hepatobiliary diseases, inflammatory bowel disease (IBD), psoriasis, and asthma, and non-immune-mediated inflammatory diseases, infectious diseases, immunodeficiency diseases, neoplasia, etc. However, there is still a need for treatments of inflammatory diseases, which are more efficient and/or exhibit fewer side effects than known treatments. The same applies for methods of preventing such diseases.

Dendritic cells are the best known integrators of external signals, they insert their dendrites within epithelial cells in Peyer's patches, just before the ileo-caecal valve, to sense microorganisms (Swiatczak B, Rescigno M. How the interplay between antigen presenting cells and microbiota tunes host immune responses in the gut. Semin Immunol. 2012 Feb;24(1):43-9). As described above, dsRNAs and potentially miRNA are likely recognized as a viral threat and therefore would be expected to elicit a pro-inflammatory response rather than the surprising anti-inflammatory response that we report.

Therefore, plant miRNA molecules targeting beneficial gene regulatory process through specified food intake (i.e probiotics, specific diet regime) will likely be sensed by DCs through specific mechanisms from the same receptors but in a different manner than viral and human miRNAs (Croce et al., 2012 PNAS) Also, DC master regulators of immune response modulated inflammation and tolerance by interacting with T cells directly or through cytokine production.

Human internally produced miRNAs are normally implicated in establishing and maintaining the cell fate of immune cells (e.g. miR-155 (O'Connell R.M. et al; MicroRNA-155 promotes autoimmune inflammation by enhancing inflammatory T cell development. Immunity. 2010), miR-181a (Li QJ et al, 2007. miR-181a is an intrinsic modulator of T cell sensitivity and selection. Cell 129:147-161), miR-150 (Xiao C et al. 2007. MiR-150 controls B cell differentiation by targeting the transcription factor c-Myb. Cell 131:146-159), miR-223 (Johnnidis JB et al., 2008. Regulation of progenitor cell proliferation and granulocyte function by microRNA-223. Nature 451:1125-1129) and miR146 (Taganov KD et al., NF-kappaB-dependent induction of microRNA miR-146, an inhibitor targeted to signaling proteins of innate immune responses. Proc Natl Acad Sci USA 2006;103:12481-6.).

Thus, in one embodiment, the present invention relates a plant sRNA extract for use as an immunosuppressive agent.

Thus, in a further embodiment, the present invention relates a plant sRNA extract for use as an anti-inflammatory agent.

Surprisingly, it was found that plant miRNA, a plant sRNA extract, and a composition comprising 2 or more plant sRNA extracts exhibit an anti-inflammatory effect, as opposed to what was observed for canonical siRNAs and exogenously supplied mammalian miRNAs (Croce et al. 2012 PNAS). Surprisingly, it could be shown that administering plant sRNA extract, a composition comprising 2 or more plant sRNA extracts of the invention and/or plant miRNA in conjunction with different inflammatory agents, dampens inflammation *per se* and also prevents inflammation.

In particular, it could be surprisingly shown that a composition comprising plant sRNA extracts of the invention ameliorate experimental autoimmune encephalomyelitis as a mouse model for multiple sclerosis (see Example 8 and Figures 21 to 24).

Therefore, the experiments surprisingly show that plant sRNA extracts, compositions comprising 2 or more plant sRNA extracts and plant miRNA are effective as anti-inflammatory agents, which can attenuate T cell proliferation and production both of inflammatory cytokines and co-stimulatory molecules in stimulated human dendritic cells, as described in the Examples.

This result shows a profound functional difference of plant miRNA as compared to human miRNA, i.e an opposite role in immuno-modulatory properties. Therefore, plant sRNA extracts and plant miRNA exhibit unique, unexpected properties.

According to the present invention, a "plant sRNA extract" is understood as a composition comprising plant material of a given plant variety wherein the amount of sRNA of said plant variety in comparison to other RNA of the same plant variety in said composition is higher than in naturally occurring plant material of said plant variety.

In a preferred embodiment, the "plant sRNA extract" is understood as a composition comprising plant material of a given plant variety wherein the amount of sRNA of said plant variety in comparison to other RNA of the same plant variety in said composition is at least 10% higher than in naturally occurring plant material of said plant variety, more preferably at least 20%, 50%, 100% or 200% higher than in naturally occurring plant material of said plant variety.

Typically, the amount is determined as (w/v) or (w/w).

In more preferred embodiment, the "plant sRNA extract" according to the present application refers to the purified small RNA fraction of a plant optionally dissolved in a suitable solvent. The plant sRNA extract when dissolved in a suitable solvent comprises less than 20% (w/v), preferably less than 10% (w/v), more preferably less than 5% (w/v), even more preferably less than less than 1% (w/v) of other plant components, in particular other RNA. A suitable solvent is preferably an aqueous solution, which is more preferably a buffered aqueous solution, which may comprise further substance like RNAse inhibitors.

In case the extract is not dissolved, the extract may be solid, for example it may be a dry, dried, or freeze-dried extract. Also, the extract may be in the form of a liquid or frozen fluid, such as a solution, suspension, dispersion, or the extract may be in the form of a gel.

For extraction from plant, a complete plant or parts thereof of may be used. Preferably, plant sRNA may be extracted from leaves, flowers, roots, fruits, seeds or parts thereof. In particular strawberry fruits may be used.

The plant or parts thereof are preferably ground or shredded prior to extraction. For example, the plant or parts thereof may be pulverized, in particular in liquid nitrogen as described in Example 1.

According to the present invention, the term "plant" encompasses any member of the plant-kingdom according to the Linnaeus definition, encompassing both vascular and non-vascular plants.

In a preferred embodiment of the extract for use of the present invention, the sRNA has a length of 200 nucleotides or less, preferably a length of 120 nucleotides or less, preferably a length between 10 and 120 nucleotides.

Therefore, in a preferred embodiment, the "sRNA plant extract" comprises the sRNA of a plant which has a length of 200 nucleotides or less, preferably a length of 120 nucleotides or less, preferably a length between 10 and 120 nucleotides.

In a more preferred embodiment "sRNA plant extract" relates to composition consisting of or essentially consisting of the sRNA of a plant which has a length of 200 nucleotides or less, preferably a length of 120 nucleotides or less, preferably a length between 10 and 120 nucleotides, preferably with the proviso that a solvent may be present in the composition.

"Essentially consisting of' is understood as that less than 10%, 5%, 1% or 0,1% of other compounds are present, preferably with the proviso that a solvent may be present in the composition.

In an even more preferred embodiment, the sRNA has a length of 10 nucleotides or more.

Therefore, in a preferred embodiment, the "sRNA plant extract" comprises the sRNA of a plant which has a length of 10 nucleotides or more.

Thus, in an even more preferred embodiment of the present invention, the sRNA of the plant sRNA extract has a length of between 10 and 200 nucleotides, preferably a length of between 10 and 160 nucleotides, more preferably a length of between 10 and 120 nucleotides, even more preferably a length of between 10 and 100 nucleotides, even more preferably a length of between 10 and 70, 10 and 60, 15 and 60, 20 an 60, or between 10 and 50 nucleotides, most preferably a length of between 10 and 25 nucleotides or between 10 and 24 nucleotides.

Therefore, in yet a further preferred embodiment, the "sRNA plant extract" or "total sRNA extract" comprises the sRNA of a plant which has a length of between 10 and 200 nucleotides, preferably a length of between 10 and 160 nucleotides, more preferably a length of between 10 and 120 nucleotides, even more preferably a length of between 10 and 100 nucleotides, even more preferably a length of between 10 and 70, 10 and 60, 15 and 60, 20 and 60, or between 10 and 50 nucleotides, most preferably a length of between 10 and 25 nucleotides. Accordingly, the sRNA plant extract preferably comprises all sRNA molecules of a plant sample which has a length of between 10 and 200 nucleotides, preferably a length of between 10 and 160 nucleotides, more preferably a length of between 10 and 120 nucleotides, even more preferably a length of between 10 and 100 nucleotides, even more preferably a length of between 10 and 70, 10 and 60, 15 and 60, 20 and 60, or between 10 and 50 nucleotides, most preferably a length of between 10 and 25 nucleotides or between 10 and 24 nucleotides, with the proviso that the sRNA plant extract optionally does not contain ribosomal RNA or optionally contains less than 70%, 50%, 10%, 10% or 1% ribosomal RNA (rRNA) of the plant sample.

The sRNA fraction extracted from plants containing the sRNA of a plant which has a length of between 10 and 25 or between 10 and 24 nucleotides corresponds to the miRNA fraction. Therefore, this plant sRNA extract is particularly preferred. The sRNA fraction extracted from plants or plant sRNA extract containing the sRNA of a plant which has a length of between 10 and 70 nucleotides, preferably 10 and 60 nucleotides, more preferably 15 and 60 nucleotides corresponds to the fraction containing miRNA and their precursors. Therefore, this sRNA plant extract is also preferred.

"sRNA" according to the present invention is small RNA, in particular RNA of a length of 200 nucleotides or less.

In a further preferred embodiment, the sRNA, in particular the sRNA of the plant sRNA extract, does not contain ribosomal RNA, or contains less than 70%, 50%, 10%, 10% or 1% ribosomal RNA (rRNA) of the same plant variety. For example, the ribosomal RNA is 5S rRNA, 5.8S rRNA, 18S rRNA, or 28S rRNA.

Therefore, in a yet further preferred embodiment, the sRNA has a length of length of 120 nucleotides or less, preferably a length between 10 and 120, 10 and 100, 10 and 70, 10 and 60, 15 and 60, 20 and 60 or 10 and 50 nucleotides.

In a preferred embodiment of the extract for use of the invention, the sRNA is miRNA.

Therefore, in a preferred embodiment, the plant sRNA extract for use is a plant miRNA extract. Accordingly, the plant miRNA extract preferably comprises all miRNA molecules of a plant sample, more preferably all miRNA which has a length of between 10 and 200 nucleotides, preferably a length of between 10 and 160 nucleotides, more preferably a length of between 10 and 120 nucleotides, even more preferably a length of between 10 and 100 nucleotides, even more preferably a length of between 10 and 70, 10 and 60, 15 and 60, 20 and 60, or between 10 and 50 nucleotides, most preferably a length of between 10 and 25 nucleotides, such as 10, 11, 12, 13, 14, 15, 16, 17, 18, 19, 20, 21, 22, 23, 24 and 25 nucleotides. The plant miRNA extract optionally does not contain ribosomal RNA or optionally contains less than 70%, 50%, 10%, 10% or 1% ribosomal RNA (rRNA) of the plant sample.

In a further embodiment, the present invention relates to plant miRNA extract for use as an immunosuppressive agent.

In a yet further embodiment, the present invention relates to plant miRNA for use as an immunosuppressive agent.

In a further embodiment, the present invention relates to a plant sRNA extract as defined above or plant miRNA for use in a method for treating and/or preventing an inflammatory disease.

As used herein the term "inflammatory disease" and/or "inflammation", used interchangeably, includes inflammatory abnormalities characterized by enhanced immune response to harmful stimuli, such as pathogens, damaged cells, or irritants. Inflammatory diseases underlie a vast variety of human diseases.

In a preferred embodiment, the inflammatory disease is selected from the group consisting of chronic prostatitis, Glomerulonephritis, Hypersensitivities, Pelvic inflammatory disease, Reperfusion injury, Sarcoidosis, Vasculitis, Interstitial cystitis, normocomplementemic urticarial vasculitis, pericarditis, myositis, anti-synthetase syndrome, scleritis, macrophage activation syndrome, Bechet's Syndrome, PAPA Syndrome, Blau's Syndrome, gout, adult and juvenile Still's disease, cryropyrinopathy, Muckle -Wells syndrome, familial cold-induced auto-inflammatory syndrome, neonatal onset multisystemic inflammatory disease, familial Mediterranean fever, chronic infantile neurologic, cutaneous and articular syndrome, systemic juvenile idiopathic arthritis, Hyper IgD syndrome, Schnitzler's syndrome, TNF receptor-associated periodic syndrome (TRAPSP), gingivitis, periodontitis, hepatitis, cirrhosis, pancreatitis, myocarditis, vasculitis, gastritis, gout, gouty arthritis, and inflammatory skin disorders, selected from the group consisting of psoriasis, atopic dermatitis, eczema, rosacea, urticaria, and acne.

In a further preferred embodiment, said inflammatory disease is an autoimmune disease.

In a particularly preferred embodiment, said autoimmune disease is selected from the group consisting of multiple sclerosis, rheumatoid arthritis; psoriatic arthritis, discoid lupus erythematosus, systemic lupus erythematosus (SLE); ulcerative colitis; Crohn's disease; benign lymphocytic angiitis, autoimmune lymphoproliferative syndrome, sarcoidosis, autoimmune thrombocytopenic purpura, idiopathic thrombocytopenic purpura, pure red cell aplasia, Sjogren's syndrome, rheumatic disease, polymyalgia rheumatica, mixed connective tissue disease, inflammatory rheumatism, degenerative rheumatism, extra-articular rheumatism, juvenile arthritis, juvenile rheumatoid arthritis, systemic juvenile idiopathic arthritis, arthritis uratica, muscular rheumatism, chronic polyarthritis, reactive arthritis, Reiter's syndrome, rheumatic fever, relapsing polychondritis, Raynaud's phenomenon, vasculitis, cryoglobulinemic vasculitis, ANCA-associated vasculitis, temporal arteritis, giant cell arteritis, Takayasu arteritis, Behcet's disease, antiphospholipid syndrome, myasthenia gravis, autoimmune haemolytic anemia, Guillain-Barre syndrome, chronic immune polyneuropathy, chronic inflammatory demyelinating polyneuropathy, autoimmune thyroiditis, insulin dependent diabetes mellitus, type I diabetes, Addison's disease, membranous glomerulonephropathy, polyglandular autoimmune syndromes, Goodpasture's disease, autoimmune gastritis, autoimmune atrophic gastritis, pernicious anemia, pemphigus, pemphigus vulgaris, cirrhosis, primary biliary cirrhosis, idiopathic pulmonary fibrosis, myositis, dermatomyositis, juvenile dermatomyositis, polymyositis, fibromyositis, myogelosis, celiac disease, celiac sprue dermatitis, immunoglobulin A nephropathy, Henoch-Schonlein purpura, Evans syndrome, atopic dermatitis, psoriasis, psoriasis vulgaris, psoriasis arthropathica, Graves' disease, Graves' ophthalmopathy, scleroderma, systemic scleroderma, progressive systemic scleroderma, diffuse scleroderma, localized scleroderma, Crest syndrome, asthma, allergic asthma, allergy, primary biliary cirrhosis, Hashimoto's thyroiditis, fibromyalgia, chronic fatigue and immune dysfunction syndrome (CFIDS), primary myxedema, sympathetic ophthalmia, autoimmune inner ear disease, autoimmune uveitis, autoimmune chronic active hepatitis, collagen diseases, ankylosing spondylitis, periarthritis humeroscapularis, panarteritis nodosa, polyarteritis nodosa, chondrocalcinosis, Wegener's granulomatosis, microscopic polyangiitis, chronic urticaria, bullous skin disorders, pemphigoid, bullous pemphigoid, cicatricial pemphigoid, vitiligo, atopic eczema, eczema, chronic urticaria, autoimmune urticaria, normocomplementemic urticarial vasculitis, hypocomplementemic urticarial vasculitis, alopecia areata, alopecia universalis, alopecia totalis, Devic's disease, pernicious anemia, childhood autoimmune hemolytic anemia, idiopathic autoimmune hemolytic anemia, refractory or chronic Autoimmune Cytopenias, Prevention of development of Autoimmune Anti-Factor VIII Antibodies in Acquired Hemophilia A, Cold agglutinin disease, Neuromyelitis Optica, Stiff Person Syndrome, gingivitis, periodontitis, pancreatitis, myocarditis, gastritis, gout, gouty arthritis, idiopathic pericarditis, anti-synthetase syndrome, scleritis, macrophage activation syndrome, PAPA Syndrome, Blau's Syndrome, adult and juvenile Still's disease, cryopyrin associated periodic syndrome, Muckle -Wells syndrome, familial cold auto- inflammatory syndrome, neonatal onset multisystem inflammatory disease, chronic infantile neurologic cutaneous and articular syndrome, familial Mediterranean fever, Hyper IgD syndrome, Schnitzler's syndrome, autoimmune retinopathy, age-related macular degeneration, and TNF receptor-associated periodic syndrome (TRAPS).

In a yet further preferred embodiment, the inflammatory disease is an allergic disease.

In a particularly preferred embodiment, said allergic disease is selected from the group consisting of
a) allergic respiratory disease, such as bronchial asthma, pediatric asthma, allergic asthma, atopic asthma, aspirin asthma, or allergic bronchitis,
b) allergic nasal disease, such as allergic rhinitis, vernal catarrh, hay fever, or chronic allergic rhinitis,
c) an allergic skin disease, such as atopic dermatitis,
d) an allergic ocular disease, such as hay fever, seasonal allergic conjunctivitis, or chronic allergic conjunctivitis,
e) hypersensitivity pneumonitis,
f) contact dermatitis, and
g) food allergy.

In a further preferred embodiment, said inflammatory disease is psoriasis.

In a further preferred embodiment, said inflammatory disease is inflammatory bowel disease, more preferably Crohn's disease or ulcerative colitis.

In a further preferred embodiment, said inflammatory disease is ulcerative colitis.

As used herein, "inflammatory bowel disease" also refers to a related disease and refers to all types and stages of inflammatory bowel disease (IBD), including, but not limited to: Crohn's disease and ulcerative colitis (UC). Optionally, conditions relating to IBD include, e.g., Collagenous colitis, Lymphocytic colitis, Ischaemic colitis, Diversion colitis, Behcet's disease, Indeterminate colitis.

As used herein, "psoriasis" also refers to a related disease and refers to all types and stages of psoriasis, including, but not limited to: Nonpustular Psoriasis including Psoriasis vulgaris and Psoriatic erythroderma (erythrodermic psoriasis), Pustular psoriasis including Generalized pustular psoriasis (pustular psoriasis of von Zumbusch), Pustulosis palmaris et plantaris (persistent palmoplantar pustulosis, pustular psoriasis of the Barber type, pustular psoriasis of the extremities), Annular pustular psoriasis, Acrodermatitis continua, Impetigo herpetiformis. Optionally, conditions relating to psoriasis include, e.g., drug-induced psoriasis, Inverse psoriasis, Napkin psoriasis, Seborrheic-like psoriasis, Guttate psoriasis, Nail psoriasis, Psoriatic arthritis.

In the Examples, it is shown that plant sRNA extracts, compositions comprising 3 plant sRNA extracts and plant miRNAs attenuate T cell proliferation and the expression and/or production of a type I IFN response, IL-1β, TNFα, CD80, CD86 and CD83 of stimulated dendritic cells. Therefore, plant sRNA extracts, compositions comprising 2 or more plant sRNA extracts and plant miRNAs are in particular useful in the treatment and/or prevention of autoimmune diseases or inflammatory diseases, in which a type I IFN response, IL-1β, TNFα, CD80, CD86 and/or CD83 play a role, and/or wherein dendritic cells are dysregulated. For example in patients with Crohn's disease, pro-inflammatory Th17 and Th1 cytokines outweigh the effect of anti-inflammatory cytokines secreted by regulatory T cells (Treg). This results in an imbalance of pro-inflammatory and anti-inflammatory cytokines. Thus, Crohn's disease may be treated and/or prevented with plant miRNA, plant sRNA extracts, and compositions comprising 2 or more plant sRNA extracts according to the present invention.

Thus, in a further preferred embodiment, the inflammatory disease is characterized by an increase in CD4⁺ T cell proliferation.

Thus, in a further preferred embodiment, the inflammatory disease is characterized by an increase in expression or production of at least one of inflammatory biomarker, in particular selected from IL-1β and TNFα and/or wherein the inflammatory disease is characterized by a dysregulation of the cytokines or co-receptors in at least, and/or by a dysregulation of immune function as detectable in changing of DC activity (i.e. costimulatory molecules expression).

Thus, in a further preferred embodiment, the inflammatory disease is characterized by an increase in expression or production of IL-10, such as lymphoma, in particular non-Hodgkin's lymphoma, and Burkitt lymphoma, melanoma and non-small cell lung cancer.

A "patient" is understood as animal, in particular a human in need of treatment and/or prevention. Accordingly, the plant sRNA extract, composition comprising 2 or more plant sRNA extracts of the invention, or plant miRNA for use in prevention and/or treatment is suitable for the prevention and/or treatment of animals, in particular humans in need of treatment and/or prevention.

As shown in the Examples, plant sRNA extracts from monocot and dicot plants are effective in attenuating the T cell proliferation. As shown in Figure 3A, a number plant sRNA extracts were shown to be effective.

Therefore, in a preferred embodiment, the plant sRNA extract is selected from an apple sRNA extract, a blueberry sRNA extract, a raspberry sRNA extract, a cabbage sRNA extract, an *Arabidopsis thaliana* sRNA extract, a grape sRNA extract, a rice sRNA extract, in particular a wild rice sRNA extract, a corn sRNA extract, a pine sRNA extract, a fern sRNA extract and a sage sRNA extract.

In a further particularly preferred embodiment, the *Arabidopsis thaliana* sRNA extract is an sRNA extract from *Arabidopsis thaliana* leaves.

In one particularly preferred embodiment, the apple sRNA extract is an sRNA extract from apple skin.

In a further particularly preferred embodiment, the cabbage sRNA extract is an sRNA extract from cabbage leaves or cabbage flower, most preferred from cabbage leaves.

In a further particularly preferred embodiment, the fern sRNA extract is an sRNA extract from fern leaves.

In a further particularly preferred embodiment, the blueberry sRNA extract is an sRNA extract from blueberry fruits.

In a further particularly preferred embodiment, the raspberry sRNA extract is an sRNA extract from raspberry fruits.

In a further particularly preferred embodiment, the grape sRNA extract is an sRNA extract from grapes.

In a further particularly preferred embodiment, the rice sRNA extract is an sRNA extract from rice grains.

In a further particularly preferred embodiment, the corn sRNA extract is an sRNA extract from corn grains.

In a further particularly preferred embodiment, the sage sRNA extract is an sRNA extract from sage leaves.

In a further particularly preferred embodiment, the pine sRNA extract is an sRNA extract from pine needle.

Surprisingly, the total sRNA extract from strawberry, namely *Fragaria vesca,* is even more effective. In a particularly preferred embodiment, the plant sRNA extract from strawberry, namely *Fragaria vesca,* is from strawberry, namely *Fragaria vesca* fruits. Therefore, in such preferred embodiment, the plant sRNA extract is a strawberry sRNA extract, more preferably a strawberry fruit sRNA extract.

Further, the total sRNA extract from strawberry, namely *Fragaria vesca,* cabbage leaves, fern leaves and apple skin was determined to be particularly effective, as shown in Figure 3B).

Therefore, in a more preferred embodiment, the plant sRNA extract is selected from an apple sRNA extract, cabbage sRNA extract, strawberry sRNA extract and fern sRNA extract.

In one particularly preferred embodiment, the apple sRNA extract is an sRNA extract from apple skin.

In a further particularly preferred embodiment, the cabbage sRNA extract is an sRNA extract from cabbage leaves or cabbage flower, most preferred from cabbage leaves.

In a further particularly preferred embodiment, the fern sRNA extract is an sRNA extract from fern leaves.

Thus, the sRNA extract of the invention may be from any plant, in particular from a monocot or dicot. For example, plant sRNA extracted from cabbage, apple skin, fern, soybean, maize, or strawberry may be used.

In a particularly preferred embodiment, the sRNA extract of the invention is from a plant selected from strawberry, namely *Fragaria vesca,* cabbage, fern and apple.

In a further particularly preferred embodiment, the sRNA extract of the invention is from a plant part selected from strawberry fruit, namely *Fragaria vesca* fruit, cabbage leaves, fern leaves and apple skin.

In a further embodiment, the present invention relates to a composition comprising 2 or more plant sRNA extracts.

In a preferred embodiment, the present invention relates to a composition consisting of 2 or more plant sRNA extracts.

In a preferred embodiment, the present invention relates to a composition comprising, preferably consisting of 2, 3, 4, 5, 6, 7, 8, 9, 10 or more plant sRNA extracts.

In a preferred embodiment, the 2, 3, 4, 5, 6, 7, 8, 9, 10 or more plant sRNA extracts are obtained from different plant families or plant varieties and/or are obtained from same plant families or plant varieties. In case, the sRNA extract is from the same plant varieties, different parts of the plant may be used, such as cabbage flowers and cabbage leaves.

In a preferred embodiment, the present invention relates to a composition comprising 2 or more plant sRNA extracts, such as 2, 3, 4, 5, 6, 7, 8, 9, 10, or 11 plant sRNA extracts, selected from an apple sRNA extract, a blueberry sRNA extract, a raspberry sRNA extract, a cabbage sRNA extract, an *Arabidopsis thaliana* sRNA extract, a grape sRNA extract, a rice sRNA extract, in particular a wild rice sRNA extract, a corn sRNA extract, a pine sRNA extract, a fern sRNA extract and a sage sRNA extract.

In a preferred embodiment, the present invention relates to a composition comprising 2 or more sRNA extracts selected from strawberry sRNA extract, namely *Fragaria vesca* sRNA extract, cabbage sRNA extract, fern sRNA extract and apple sRNA extract.

In a further embodiment, the present invention relates to a composition comprising, preferably consisting of 2, 3, or 4 sRNA extracts selected from strawberry sRNA extract, namely *Fragaria vesca* extract, cabbage extract, fern extract and apple extract, more preferably the composition comprises, preferably consists of 2, 3, or 4 sRNA extracts selected from strawberry fruit sRNA extract, namely *Fragaria vesca* fruit extract, cabbage leaves extract, fern leaves extract and apple skin extract.

Therefore, in a further embodiment, the present invention relates to a composition comprising, preferably consisting of, 2, 3, or 4 sRNA extracts selected from strawberry sRNA extract, namely *Fragaria vesca* extract, cabbage extract, fern extract and apple extract, more preferably the composition comprises, preferably consists of 2, 3, or 4 sRNA extracts selected from strawberry fruit sRNA extract, namely *Fragaria vesca* fruit extract, cabbage leaves extract, fern leaves extract and apple skin extract.

The compositions of the present invention can be obtained by methods known to the skilled person, e.g. by preparing the individual plant sRNA extracts, as described above, and by mixing the individual plant sRNA extracts. For example, a composition comprising 3 different plant sRNA extracts is prepared in Example 1, namely a combination of sRNA plant extracts from fern leaves, cabbage leaves and apple skin, referred in the Examples as "pool".

Surprisingly, the "pool" composition was successfully used in the EAE mouse model for multiple sclerosis.

The compositions of the present invention can be used for the medical indications as described herein, e.g. as an immunosuppressive agent, and for treating and/or preventing an inflammatory disease as described herein, in particular wherein the inflammatory disease is an autoimmune disease, more preferably wherein the inflammatory disease is multiple sclerosis.

In a preferred embodiment, the present invention relates to composition comprising, preferably consisting of 2 or more sRNA extracts selected from strawberry sRNA extract, namely *Fragaria vesca* sRNA extract, cabbage sRNA extract, fern sRNA extract and apple sRNA extract, more preferably the composition comprises, preferably consists of 2, 3, or 4 sRNA extracts selected from strawberry fruit sRNA extract, namely *Fragaria vesca* fruit sRNA extract, cabbage leaves sRNA extract, fern leaves sRNA extract and apple skin sRNA extract.

In a preferred embodiment, the present invention relates to a composition comprising, preferably consisting of cabbage sRNA extract, a fern sRNA extract and a apple sRNA extract, and optionally strawberry sRNA extract, namely *Fragaria vesca* sRNA extract, more preferably the composition comprises, preferably consists of a cabbage leaves sRNA extract, fern leaves sRNA extract and apple skin sRNA extract, and optionally strawberry fruit sRNA extract, namely *Fragaria vesca* fruit sRNA extract.

In a preferred embodiment, the sRNA extracts of a composition of the invention each comprise all sRNA molecules of the respective plant sample which has a length of between 10 and 200 nucleotides, preferably a length of between 10 and 160 nucleotides, more preferably a length of between 10 and 120 nucleotides, even more preferably a length of between 10 and 100 nucleotides, even more preferably a length of between 10 and 70, 10 and 60, 15 and 60, 20 and 60, or between 10 and 50 nucleotides, most preferably a length of between 10 and 25 nucleotides or between 10 and 24 nucleotides, with the proviso that the sRNA plant extract optionally does not contain ribosomal RNA or optionally contains less than 70%, 50%, 10%, 10% or 1% ribosomal RNA (rRNA) of the plant sample.

In a further preferred embodiment, the composition of the invention does not contain further plant sRNA extracts, in particular no further plant sRNA extracts from other plants.

In a further preferred embodiment, the present invention relates to a composition consisting of cabbage leaves sRNA extract, fern leaves sRNA extract and apple skin sRNA extract, preferably, wherein the composition contains (1) 5 to 90 % cabbage leaves sRNA extract (w/w), (2) 5 to 90% fern leaves sRNA extract (w/w) and (3) 5 to 90% apple skin sRNA extract (w/w), preferably (1) 20 to 40 % cabbage leaves sRNA extract (w/w), (2) 20 to 40% fern leaves sRNA extract (w/w) and (3) 20 to 40% apple skin sRNA extract (w/w).

It is understood that the sum of (1) +(2)+(3) is 100%.

In a preferred embodiment of the present invention, the composition is a solution, in particular a buffered solution, such as a buffered saline solution, and/or wherein the composition optionally further comprises pharmaceutically acceptable carriers and/or edible auxiliary agents.

In one preferred embodiment, the composition is a pharmaceutically acceptable solution, in particular a solution suitable for intravenous injection.

In another preferred embodiment, the composition is an edible composition, in particular a composition edible for humans.

It is understood that the preferred embodiment for the plant sRNA extracts according to the present invention also apply to the compositions comprising 2 or more plant sRNA extracts of the invention and composition comprising 2 or more plant sRNA extracts for use of the invention as well as to the methods related thereto.

In a yet further embodiment, the present invention relates to a plant sRNA extract as disclosed herein or a composition comprising 2 or more plant sRNA extracts according to the present invention, for use as a medicament.

The preferred embodiments disclosed herein for plant sRNA extracts and compositions comprising 2 or more plant sRNA extracts of the invention also apply to the plant sRNA extracts and compositions comprising 2 or more plant sRNA extracts for use as a medicament.

In a further embodiment the present invention relates to a composition comprising 2 or more plant sRNA extracts of the invention for use as an immunosuppressive agent.

The present invention relates in a yet further embodiment to a composition comprising 2 or more plant sRNA extracts of the invention for use in a method for treating and/or preventing an inflammatory disease.

For example, it could surprisingly be shown in the examples that an amelioration of the disease could be achieved in the EAE mouse model for multiple sclerosis using a composition comprising fern sRNA extract, apple sRNA extract and cabbage sRNA extract.

In a preferred embodiment of the plant sRNA extract or plant miRNA or plant miRNA extract for use of the invention, the plant is a land plant (*Embriophytae*)*,* such as a flowering plant, fern or pine.

In a more preferred embodiment of the plant sRNA extract or plant miRNA or plant miRNA extract for use of the invention, the plant is a vascular plant (*Tracheophytes*)*,* such as a fern.

In one further preferred embodiment of the plant sRNA extract or plant miRNA or plant miRNA extract for use of the invention, the plant is a fern (*Pteridophyta*)*.*

In one further preferred embodiment of the plant sRNA extract or plant miRNA or plant miRNA extract for use of the invention, the plant is a seed plant (*Spermatophytes*)*,* such as a flowering plant or pine.

In a preferred embodiment of the plant sRNA extract or plant miRNA or plant miRNA extract for use of the invention, the plant is selected from apple, blueberry, raspberry, strawberry, cabbage, *Arabidopsis thaliana,* grape (or *Vitis vinifera*)*,* rice, such as *Oryza sativa* or wild rice, corn, such as *Zea mays,* pine, fern and sage.

In a preferred embodiment of the plant sRNA extract or plant miRNA or plant miRNA extract for use of the invention, the plant is a flowering plant (*Magnoliophyta*)*.*

In a more preferred embodiment of the plant sRNA extract or plant miRNA for use of the invention, the plant is a *Rosacea spp.*

*Rosaceae* plants are known to a skilled person in include the subfamily *Rosoideae,* in particular rose, blackberry, raspberry, strawberry, Potentilla and Geum; the subfamily *Amygdaloideae,* in particular pome fruits, traditionally known as subfamily *Maloideae* or *Pyroideae,* like apple, cotoneaster, and hawthorn; the *Spiraeoideae;* and the subfamily *Dryadoideae,* in particular the genera *Dryas, Cercocarpus, Chamaebatia, Cowania,* and *Purshia.*

In an even more preferred embodiment of the plant sRNA extract or plant miRNA for use of the invention, the plant is a strawberry, most preferred *Fragaria vesca.* Accordingly, in an even more preferred embodiment of the plant sRNA extract or plant miRNA for use of the invention, the plant sRNA extract is derived from strawberry, most preferred from *Fragaria vesca.*

In a yet further preferred embodiment, the composition of the invention comprises a strawberry sRNA extract.

In another preferred embodiment of the plant sRNA extract or plant miRNA or plant miRNA extract for use of the invention, the plant is a conifer *(Pinophyta),* such as a pine.

In a yet further preferred embodiment, one or more different plant sRNA extracts and/or plant miRNAs are administered. For example 2, 3, 4, 5, 6, 7, 8, 9 or 10 different plant sRNA extracts may be administered. For example, sRNA extracts from strawberry and soybean may be administered.

The one or more different plant sRNA extracts may be administered together or separately, or they may be administered simultaneously, or at different time points. In case the different plant sRNA extracts are administered together, a composition comprising 2 or more plant sRNA extracts of the invention may be administered.

Moreover, it could be shown that synthetic specific plant miRNAs are effective in attenuating T cell proliferation and the expression and/or production of inflammatory cytokines IL-1β, TNFα, and immune reactivity of stimulated dendritic cells by means of CD80, CD86 and CD83 proper expression. A synthetic miRNA is understood as a miRNA which exists naturally in a plant, which is obtained by chemical synthesis. Alternatively, the specific plant miRNAs may be purified from plants. The synthesis of miRNA is known in the art and is for example described in Example 1.

In a further embodiment, the plant miRNA for use of the invention is preferably a purified miRNA.

A purified plant miRNA typically contains less than 10%, 1%, 0,01% or 0,001% of other components, in particular plant components.

The one or more different plant miRNAs may be administered together or separately, or they may be administered simultaneously, or at different time points.

Preferably, at least one plant sRNA extract and/or two or more purified miRNAs are administered.

Surprisingly, miR168 from *Fragaria vesca* is highly effective regarding its immunosuppressive effect, as shown in the examples. Thus, in a further preferred embodiment, the plant miRNA for use according to the invention is *F. vesca* miR168.

In particular, plant sRNA extracts from one plant or more than one plant may be administered. For example plant sRNA extracts from two different plant varieties or different plant families may be administered. For example, a composition comprising 2 or more plant sRNA extracts from two different plant varieties or different plant families of the invention may be administered.

"More than one" is understood to encompass for example 2, 3, 4, 5, 6, 7, 8, 9, 10, 11, 12, 13, 14, 14, 20, 40 or more.

Plant sRNA extracts can be obtained as described above and in the Examples.

In a further preferred embodiment, the two or more plant sRNA extracts may be administered as a mixture of plant sRNA extracts. In particular, plant sRNA extracts from two different plant varieties or different plant families may be mixed and may be administered thereafter. Thereby, compositions of the invention comprising two or more plant sRNA extracts, as described above, are obtained.

The plant sRNA extracts preferably contain methylated plant miRNAs or the plant miRNAs of the extract are methylated. In a further preferred embodiment, a plant miRNA for use is a methylated plant miRNA. A methylated plant miRNA for use according to the invention is preferably methylated at the 2'OH group of the 3' terminus.

Therefore, in a particularly preferred embodiment, the plant sRNA, in particular plant miRNA, of the plant sRNA extracts or mixtures thereof, is methylated sRNA, preferably methylated miRNA, more preferably sRNA or miRNA methylated at the 2'OH group of the 3' terminus.

According to a preferred embodiment of the plant sRNA extract for use of the invention, or the composition of the invention comprising 2 or more plant sRNA extracts, the sRNA molecules of the extract, and/or the the sRNA molecules of the composition comprise or consist of methylated sRNA, more preferably sRNA or miRNA methylated at the 2'OH group of the 3' terminus.

A plant sRNA extract or a composition comprising 2 or more plant sRNA extracts may contain both mature sRNA molecules and precursor RNA molecules thereof.

The miRNAs for use according to the invention may be produced recombinantly using miRNA expression constructs, using methods known in the art (Schwab, R., Ossowski, S., Riester, M., Warthmann, N., and Weigel, D. (2006). Highly specific gene silencing by artificial microRNAs in Arabidopsis. The Plant Cell 18, 1121-1133), or may be isolated from plants.

Preferably, mature miRNA molecules are administered.

A number of miRNAs are described in the prior art and may be used according to the invention. These miRNAs are described in a large number of plant species as shown in Table 1 below (Kozomara, A., and Griffiths-Jones, S. (2011). miRBase: integrating microRNA annotation and deep-sequencing data. Nucleic Acids Research 39, D152-D157). These miRNAs were also described in *Fragaria ananassa* (Ge, A., Shangguan, L., Zhang, X., Dong, Q., Han, J., Liu, H., Wang, X., and Fang, J. (2012). Deep sequencing discovery of novel and conserved microRNAs in strawberry (Fragaria×ananassa). Physiologia Plantarum).

**Table 1:**

| miRNA | Target sequence | Cleaved Genes |
|---|---|---|
| miR156/157 (12) | SQUAMOSA-promoter binding | *SPL2, SPL3, SPL10* |
| miR158 (2) | Unknown | |
| miR159 (6) | MYB, TCP | *MYB33, MYB65, TCP2, TCP3, TCP4,TCP10, TCP24* |
| miR160 (3) | ARF | *ARF10, ARF17* |
| miR161 (1) | Pentatricopeptide repeat | *At1g06580* |
| miR162 (2) | Dicer | *DCL1* |
| miR163 (1) | Methyl transferases | |
| miR164 (3) | NAC-domain transcription factor | *CUC1, CUC2, NAC1, At5g07680, At5g61430* |
| miR165/166 (9) | HD-ZIP transcription factor | *PHB, PHV, REV, C3HDZIP1* |
| miR167 (4) | ARF | *ARF8* |
| miR168 (2) | AGO | *AGO1* |
| miR169 (14) | CCAAT-binding factor HAP2-like protein | *At3g05690* |
| miR170/171 (4) | GRAS-domain transcription factor | *SCL6-III, SCL6-IV* |
| miR172 (5) | APETALA2-like transcription factor | *AP2, TOE1, TOE2, TOE3* |
| miR173 (1) | Unknown | |
| miR393 (2) | Auxin receptors | *TIR1, AFB1, AFB2, AFB3* |
| miR394 (2) | F-box protein | *Atlg27340* |
| miR395 (6) | ATP sulfurylase | *APS4* |
| miR396 (2) | Growth-regulating-factor transcription factor Rhodenase-like protein, Kinesin-like protein | *GRL1, GRL2, GRL3, GRL7, GRL8, GRL9* |
| miR397 (2) | Laccase, Beta-6 tubulin | *At2g29130, At2g38080, At5g60020* |
| miR398 (3) | CSD, Cytochrome C oxidase subunit V | *CSD1, CSD2, At3g15640* |
| miR399 (6) | Phosphate transporter | |

Preferably, miR156, miR168, osamiR168, or mixtures thereof are used.

A particularly preferred miRNA is miR168, in particular *Fragaria vesca* miR168.

A "miRNA" or "microRNA" for use according to the present invention is understood as oligoribonucleic acid, of about 19 to 24 nucleotides (nt) in length, which regulates expression of a polynucleotide comprising a target sequence. miRNAs are non-protein-coding RNAs and have been identified in both animals and plants. miRNAs are derived in plants via Dicer-like 1 processing of larger precursor polynucleotides. The term "miRNA" also encompasses "artificial miRNA", which comprise a miRNA sequence that is synthetically designed to silence a target sequence. In particular, such artificial miRNA exhibits 80% or more, preferably 90% or more, even more preferably 91%, 92%, 93%, 94%, 95%, 96%, 97%, 98%, 99% or more sequence identity with the nucleotide sequence of the unmodified miRNA or miRNA precursor backbone. In a preferred embodiment, the plant miRNA is methylated at the 2'OH group at the 3' terminus.

In a preferred embodiment, endogenous miRNAs are used, as the mechanism appears to be target-independent.

"miRNA precursor backbone" is a polynucleotide that provides the backbone structure necessary to form a hairpin RNA structure which allows for the processing and ultimate formation of the miRNA. Preferably, the backbone is from strawberry, rice, maize or soybean. microRNA precursor backbones have been described previously. For example, US20090155910A1 (WO 2009/079532) discloses the following soybean miRNA precursor backbones: 156c, 159, 166b, 168c, 396b and 398b, and US 20090155909A1(WO 2009/ 079548) discloses the following maize miRNA precursor backbones: 159c, 164h, 168a, 169r, and 396h. Examples of miRNA precursor backbones include the miRNA GM-396b precursor backbone or active variants thereof and the miRNA GM-159 precursor backbone or active variants thereof, as described in WO 2012/154824.

A "miRNA expression construct" is understood as DNA construct which comprises a miRNA precursor backbone having a polynucleotide sequence encoding a miRNA and a star sequence. Such miRNA expression construct may be in a suitable vector, to allow expression in a suitable host, followed by isolation of the miRNA. Such expression systems are known to a skilled person and are for example described in (Schwab, R., Ossowski, S., Riester, M., Warthmann, N., and Weigel, D. (2006). Highly specific gene silencing by artificial microRNAs in Arabidopsis. The Plant Cell 18, 1121-1133)

The plant miRNAs and plant sRNA extracts for use and compositions comprising 2 or more plant sRNA extracts surprisingly exhibit anti-inflammatory activity. Such effect may be enhanced by combining the plant miRNAs, and plant sRNA extracts or compositions comprising 2 or more plant sRNA extracts with other known anti-inflammatory agents.

Thus, in a further preferred embodiment, the plant sRNA extract or the plant miRNA for use of the invention, or composition comprising 2 or more plant sRNA extracts of the invention is administered in combination with at least one other anti-inflammatory agent.

In a more preferred embodiment, said other anti-inflammatory agent is selected from the group consisting of a corticosteroid, a statin, interferon beta, a nonsteroidal anti-inflammatory drug (NSAID), methotrexate, Cyclosporin A and a disease-modifying anti-rheumatic drug (DMARD).

In another embodiment, the plant sRNA extract or the plant miRNA for use according to the invention, or composition comprising 2 or more plant sRNA extracts of the invention is comprised in a pharmaceutical composition.

Thus, in a further embodiment, the present invention relates to a pharmaceutical composition comprising at least one plant miRNA or plant sRNA extract or composition comprising 2 or more plant sRNA extracts as defined herein, for use in a method for treating and/or preventing an inflammatory disease, as defined above.

In a preferred embodiment, the pharmaceutical composition comprises at least one plant miRNA or plant sRNA extract, or a composition comprising 2 or more plant sRNA extracts and at least one carrier. Carriers are generally known to those skilled in the art and include saline, sugars, polypeptides, polymers, lipids, creams, gels, micelle materials, and metal nanoparticles. In one more preferred embodiment, the carrier comprises at least one of the following: water, a glucose solution, a polycationic binding agent, a cationic lipid, a cationic micelle, a cationic polypeptide, a hydrophilic polymer grafted polymer, a non-natural cationic polymer, a cationic polyacetal, a hydrophilic polymer grafted polyacetal, a liposome, a ligand functionalized cationic polymer, a ligand functionalized-hydrophilic polymer grafted polymer, and a ligand functionalized liposome. In another more preferred embodiment, the polymers comprise a biodegradable histidine-lysine polymer, a biodegradable polyester, such as poly(lactic acid) (PLA), poly(glycolic acid) (PGA), and poly(lactic-co-glycolic acid) (PLGA), a polyamidoamine (PAMAM) dendrimer, a cationic lipid, or a PEGylated PEL.

In a yet further embodiment, the present invention relates to a pharmaceutical composition, food additive or dietary supplement, comprising a composition comprising 2 or more plant sRNA extracts of the invention, or a plant sRNA extract for use of the invention, preferably wherein the pharmaceutical composition, food additive or dietary supplement comprises a) lipids, more preferably at least one liposome, or b) an exosome.

In one preferred embodiment, the pharmaceutical composition, food additive or dietary supplement comprises lipids, in particular at least one lipid, at least two lipids, at least three lipids or at least four lipids, such as 1, 2, 3, 4 or 5 lipids. In a further preferred embodiment, the pharmaceutical composition, food additive or dietary supplement comprises cationic lipids, in particular 1, 2, 3, 4 or 5 cationic lipids.

In a preferred embodiment, the lipids are capable of forming a liposome. In particular, cationic lipids are suitable for this purpose.

Cationic lipids preferably include DOTAP, DOPE, DC-Chol/DOPE, DOTMA, and DOTMA/DOPE.

In certain embodiments, a pharmaceutical composition, food additive or dietary supplement of the invention comprises a cationic lipid, a neutral lipid, a sterol, and/or a disaggregation lipid.

In certain embodiments, a sterol is cholesterol.

In certain embodiments, a disaggregation lipid is a polyethylene glycol-modified lipid (PEG-modified lipid). In certain embodiments a PEG-modified lipid is PEG-dimyristoyl glycerol (PEG-DMG). In certain embodiments a PEG-modified lipid is PEG-distyryl glycerol (PEG-DSG). In certain embodiments a PEG-modified lipid is PEG-carbamoyl-1,2-dimyristyloxypropylamine (PEG-cDMA).

In certain embodiments, a neutral lipid is a phospholipid. In certain embodiments, a phospholipid is selected from phosphatidylcholine (PC), distearoylphosphatidylcholine (DSPC), and dipalmitoylphosphatidylcholine (DPPC).

In certain embodiments, the pharmaceutical composition, food additive or dietary supplement consists of a cationic lipid, and a plant sRNA extract or a composition comprising 2 or more plant sRNA extracts of the invention or plant miRNA for use of the invention.

In certain embodiments, the ratio of total lipid to sRNA is from 5 to 35 (i.e. from 5 to 1 to 35 to 1, lipid weight to sRNA weight). In certain embodiments, the ratio of total lipid to sRNA is from 5 to 15 (i.e. from 5 to 1 to 15 to 1, lipid weight to sRNA weight).

In certain embodiments, a pharmaceutical composition, food additive or dietary supplement, of the invention comprises or consists of a lipid, and an aqueous component; the aqueous component comprises plant sRNA extract(s) or a composition comprising 2 or more plant sRNA extracts according to the invention in an aqueous medium. The aqueous medium is preferably a pharmaceutically acceptable aqueous solution, such as a pharmaceutically acceptable aqueous buffered solution. The aqueous component may be a solution, dispersion, suspension or hydrogel.

Disaggregation Lipids: Examples of lipids that reduce aggregation of particles during formation include polyethylene glycol (PEG)-modified lipids, monosialoganglioside Gml, and polyamide oligomers ("PAO") such as described in U.S. Pat. No. 6,320,017. Other compounds with uncharged, hydrophilic, steric-barrier moieties, which prevent aggregation during formulation, like PEG, Gml or ATTA, can also be coupled to lipids for use as in the methods and compositions of the invention. ATTA-lipids are described, e.g., in U.S. Pat. No. 6,320,017, and PEG-lipid conjugates are described, e.g., in U.S. Pat. Nos. 5,820,873, 5,534,499 and 5,885,613. Typically, the concentration of the lipid component selected to reduce aggregation is about 1 to 15% by mole percent of lipids.

Specific examples of PEG-modified lipids (or lipid-polyoxyethylene conjugates) that are useful can have a variety of "anchoring" lipid portions to secure the PEG portion to the surface of the lipid vesicle. Examples of suitable PEG-modified lipids include PEG-modified phosphatidylethanolamine and phosphatidic acid, PEG-ceramide conjugates (e.g., PEG-CerC14 or PEG-CerC20) which are described in U.S. Ser. No. 08/486,214, PEG-modified dialkylamines and PEG-modified 1,2-diacyloxypropan-3-amines. Particularly suitable are PEG-modified diacylglycerols and dialkylglycerols.

In embodiments where a sterically-large moiety such as PEG or ATTA are conjugated to a lipid anchor, the selection of the lipid anchor depends on what type of association the conjugate is to have with the lipid particle. It is well known that mPEG (mw2000)-diastearoylphosphatidylethanolamine (PEG-DSPE) will remain associated with a liposome until the particle is cleared from the circulation, possibly a matter of days. Other conjugates, such as PEG-CerC20 have similar staying capacity. PEG-CerC14, however, rapidly exchanges out of the formulation upon exposure to serum, with a T1/2 less than 60 minutes in some assays. As illustrated in U.S. patent application Ser. No. 08/486,214, at least three characteristics influence the rate of exchange: length of acyl chain, saturation of acyl chain, and size of the steric-barrier head group. Compounds having suitable variations of these features may be useful for the invention. For some therapeutic applications it may be suitable for the PEG-modified lipid to be rapidly lost from the nucleic acid-lipid particle in vivo and hence the PEG-modified lipid will possess relatively short lipid anchors. In other therapeutic applications it may be suitable for the nucleic acid-lipid particle to exhibit a longer plasma circulation lifetime and hence the PEG-modified lipid will possess relatively longer lipid anchors.

It should be noted that aggregation preventing compounds do not necessarily require lipid conjugation to function properly. Free PEG or free ATTA in solution may be sufficient to prevent aggregation. If the particles are stable after formulation, the PEG or ATTA can be dialyzed away before administration to a subject.

Neutral Lipids: Neutral lipids, when present in the liposome, can be any of a number of lipid species which exist either in an uncharged or neutral zwitterionic form at physiological pH. Such lipids include, for example diacylphosphatidylcholine, diacylphosphatidylethanolamine, ceramide, sphingomyelin, dihydrosphingomyelin, cephalin, and cerebrosides. The selection of neutral lipids for use in the particles described herein is generally guided by consideration of, e.g., liposome size and stability of the liposomes in the bloodstream. Suitably, the neutral lipid component is a lipid having two acyl groups, (i.e., diacylphosphatidylcholine and diacylphosphatidylethanolamine). Lipids having a variety of acyl chain groups of varying chain length and degree of saturation are available or may be isolated or synthesized by well-known techniques. In one group of embodiments, lipids containing saturated fatty acids with carbon chain lengths in the range of C10 to C20 are suitable. In another group of embodiments, lipids with mono- or di-unsaturated fatty acids with carbon chain lengths in the range of C10 to C20 are used. Additionally, lipids having mixtures of saturated and unsaturated fatty acid chains can be used.

In certain embodiments, the neutral lipids used in the present invention include but are not limited to phosphatidylcholine (PC), 1,2-Dioleoyl-sn-glycero-3-phosphocholine (DOPC), lecithin, phosphatidylethanolamine (PE), lysolecithin, lysophosphatidylethanolamine, sphinogomyelin (SM), cardiolipin, phosphosphatidic acid, 1,2-Distearoyl-sn-glycero-3-phosphocholine (DSPC), 1,2-Dipalmitoyl-sn-glycero-3-phosphoethanolamine (DPPE), 1-Palmitoyl-2-oleoyl-sn-glycero-3-phosphocholine (POPC), 1,2-Dilauroyl-sn-glycero-3-phosphocholine (DLPC), 1,2-Dimyristoyi-sn-glycero-3-phosphocholine (DMPC), 1,2-Dipalmitoyl-sn-glycero-3-phosphocholine (DPPC), 1,2-Dimyristoyl-sn-glycero-3- phosphoethanolamine (DMPE), 1,2-Dioleoyl-sn-glycero-3-phosphoethanolamine (DOPE), dipalmitoloeoyl-PE, diphytanoyl-PE, DSPE, dielaidoyl-PE, dilinoleoyl-SM, and dilinoleoyl-PE. The neutral lipids useful in the present invention may also be composed of sphingomyelin, dihydrosphingomyeline, or phospholipids with other head groups, such as serine and inositol. In certain embodiments, neutral lipids are DOPE, DSPC, POPC, DPPC or any related phosphatidylcholine.

The sterol component, when present, can be any of those sterols conventionally used in the field of liposome, lipid vesicle or lipid particle preparation. In certain embodiments, the sterol is cholesterol.

Cationic Lipids: Cationic lipids carry a net positive charge at about physiological pH. Suitable cationic lipids include, but are not limited to, N,N-dioleyl-N,N-dimethylammonium chloride ("DODAC"); N-(2,3-dioleyloxyl)propyl-N,N-N-triethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP"); 1,2-Dioleyloxy-3-trimethylaminopropane chloride salt ("DOTAP.CI"); 3β-(N-(N',N'-dimethylaminoethane)-carbamoyl)cholesterol ("DC-Chol"), N-(1-(2,3-dioleyloxyl)propyl)-N-2-(sperminecarboxamido)ethyl)-N,N-dimethylammonium trifluoracetate ("DOSPA"), dioctadecylamidoglycyl carboxyspermine ("DOGS"), 1,2-dileoyl-sn-3-phosphoethanolamine ("DOPE"), 1,2-dioleoyl-3-dimethylammonium propane ("DODAP"), N, N-dimethyl-2,3-dioleyloxy)propylamine ("DODMA"), and N-(1,2-dimyristyloxyprop-3-yl)-N,N-dimethyl-N-hydroxyethyl ammonium bromide ("DMRIE"). Additionally, a number of commercial preparations of cationic lipids can be used, such as, e.g., LIPOFECTIN (including DOTMA and DOPE, available from GIBCO/BRL), and LIPOFECTAMINE (comprising DOSPA and DOPE, available from GIBCO/BRL). In particular embodiments, a cationic lipid is an amino lipid.

Additional cationic lipids include 1,2-dilinoleyloxy-3-dimethylaminopropane (DLinDMA) 1,2-Dilinoleoyl-3-dimethylaminopropane (DLinDAP), 1-Linoleoyl-2-linoeyloxy-3-dimethylaminopropane (DLin-2-DMAP), 1,2-Dilinoleylcarbamoyloxy-3-dimethylaminopropane (DLin-C-DAP), 1,2-Dilinoleylthio-3-dimethylaminopropane (DLin-S-DMA), 2,2-Dilinoleyl-4-dimethylaminomethyl-[1,3]-dioxolane (DLin-K-DMA).

Anionic lipids suitable for use in compositions of the present invention include, but are not limited to, phosphatidylglycerol, cardiolipin, diacylphosphatidylserine, diacylphosphatidic acid, N-dodecanoyl phosphatidylethanoloamine, N-succinyl phosphatidylethanolamine, N-glutaryl phosphatidylethanolamine, lysylphosphatidylglycerol, and other anionic modifying groups joined to neutral lipids.

In certain embodiments, pharmaceutical compositions, food additives or dietary supplements of the invention include amphipathic lipids. "Amphipathic lipids" refer to any suitable material, wherein the hydrophobic portion of the lipid material orients into a hydrophobic phase, while the hydrophilic portion orients toward the aqueous phase. Such compounds include, but are not limited to, phospholipids, aminolipids, and sphingolipids. Representative phospholipids include sphingomyelin, phosphatidylcholine, phosphatidylethanolamine, phosphatidylserine, phosphatidylinositol, phosphatidic acid, palmitoyloleoyl phosphatdylcholine, lysophosphatidylcholine, lysophosphatidylethanolamine, dipalmitoylphosphatidylcholine, dioleoylphosphatidylcholine, distearoylphosphatidylcholine, or dilinoleoylphosphatidylcholine. Other phosphorus-lacking compounds, such as sphingolipids, glycosphingolipid families, diacylglycerols, and α-acyloxyacids, can also be used. Additionally, such amphipathic lipids can be readily mixed with other lipids, such as triglycerides and sterols.

In a yet further preferred embodiment, exosomes may be used. Such exosomes and their preparation are described e.g. in Montecalvo et al. (2012, Blood, 119: 756-766, and Stoorvogel, 2012, Blood, 119: 646-648).

For example, exosomes loaded with sRNAs of the plant sRNA extracts or compositions comprising 2 or more plant sRNA extracts may be used. For example, exosomes loaded with plant miRNA may be used.

Further suitable pharmaceutical carriers are e.g. described in Remington's Pharmaceutical Sciences, supra, a standard reference text in this field. The pharmaceutical composition may further comprise conventional pharmaceutical additives and adjuvants, excipients or diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers, and the like.

A particularly preferred carrier or lipid is DOTAP, as used in the Examples.

Using DOTAP, liposomes are formed which enable protected delivery of the plant sRNA extracts and compositions comprising 2 or more plant sRNA extracts as described herein.

For example, it could surprisingly be shown in the examples that an amelioration of the disease could be achieved in the EAE mouse model for multiple sclerosis using a composition comprising fern sRNA extract, apple sRNA extract and cabbage sRNA extract, and DOTAP as carrier.

Preferably, the pharmaceutical composition comprises at least one pharmaceutically acceptable RNAse inhibitor.

In still another embodiment, the carrier is a polymer, e.g. a histidine-lysine copolymer that forms a nanoparticle with the miRNA molecule(s). The diameter of the nanoparticle is about typically 100 nm to about 1000 nm.

The invention thus also provides a nanoparticle comprising one or more of the plant miRNA or plant sRNA extract for use of the invention or composition comprising 2 or more plant sRNA extracts of the invention. In an embodiment, the nanoparticle further comprises a carrier, such as one or more of those described herein, and optionally a targeting ligand.

In one further embodiment of the present invention, said plant miRNA or plant sRNA extract or composition comprising 2 or more plant sRNA extracts is comprised in a food additive or dietary supplement. Preferably, the dietary supplement, food additive, food or foodstuff comprises at least one RNAse inhibitor which is acceptable for such compositions. In particular, such RNAse inhibitor is not toxic.

Methods for determining the amount of sRNA or specific miRNA are known in the art. For example chip-based detection assays, like Agilent small RNA assays, or Millipore SmartRNAplex™ miRNA profiling assay, or PCR-based detection assays, like Qiagen miScript™ PCR System, TaqMan® MicroRNA Assays, or Exiqon miRCURY LNA™ Detection may be used.

Such food additive may be added to a food product, foodstuff, dietary supplement, nutritional supplement or a supplement composition for a food product or a foodstuff, for example beverages (e.g. but not limited to sports beverages, functional waters, juices, smoothies; instant drinks), soups, dairy products (e.g. but not limited to single shot yoghurt drinks), nutritional bars, and spreads, in particular beverages and nutritional bars.

For example, a plant sRNA extract, such as a *Fragaria vesca* sRNA extract, or a composition comprising 2 or more plant sRNA extracts, such as fern sRNA extract, cabbage sRNA extract and apple sRNA extract (see example 1) may be prepared in liquid form such as an aqueous solution which optionally contains at least one RNAse inhibitor. Such solution can be used as food additive e.g. for beverages or dairy products. As used herein, the term food product refers to any food or feed suitable for consumption by humans or animals. The food product may be a prepared and packaged food (e.g., mayonnaise, salad dressing, bread, or cheese food) or an animal feed (e.g., extruded and pelleted animal feed, coarse mixed feed or pet food composition). As used herein, the term foodstuff refers to any substance fit for human or animal consumption. The term dietary supplement refers to a small amount of a compound for supplementation of a human or animal diet packaged in single or multiple dose units. Dietary supplements do not generally provide significant amounts of calories but may contain other micronutrients (e.g., vitamins or minerals). The term nutritional supplement refers to a composition comprising a dietary supplement in combination with a source of calories.

Food products or foodstuffs are for example beverages such as non-alcoholic and alcoholic drinks as well as liquid preparation to be added to drinking water and liquid food, non-alcoholic drinks are for instance soft drinks, sport drinks, fruit juices, such as for example orange juice, apple juice and grapefruit juice; lemonades, teas, near-water drinks and milk and other dairy drinks such as for example yoghurt drinks, and diet drinks. In another embodiment food products or foodstuffs refer to solid or semi-solid foods comprising the composition according to the invention. These forms can include, but are not limited to baked goods such as cakes and cookies, puddings, dairy products, confections, snack foods, or frozen confections or novelties (e.g., ice cream, milk shakes), prepared frozen meals, candy, snack products (e.g., chips), liquid food such as soups, spreads, sauces, salad dressings, prepared meat products, cheese, yoghurt and any other fat or oil containing foods, and food ingredients (e.g., wheat flour). The term food products or foodstuffs also includes functional foods and prepared food products, the latter referring to any prepackaged food approved for human consumption.

The food products or foodstuffs may already contain RNA prior to the addition of a food additive comprising a plant miRNA or a plant sRNA extract for use according to the invention or a composition comprising 2 or more plant sRNA extracts of the invention. This may be the case for food products or foodstuff being, containing or derived from plants or parts thereof, such as fruits, or fruit juices.

In this embodiment, the resulting food products or foodstuffs contain an enlarged amount and/or concentration of plant miRNA or plant sRNA extract(s) compared to the food products or foodstuffs without addition of such food additive.

In a preferred embodiment, such food products or foodstuffs contain an amount of plant sRNA which is at least 10% higher than the amount in the food product or foodstuff without addition of such food additive comprising a plant sRNA extract, more preferably the amount is at least 20%, 50%, 100% or 200% higher than in the food product or foodstuff without addition of such food additive comprising a plant sRNA extract.

In a further preferred embodiment, such food products or foodstuffs contain an amount of a plant sRNA which is at least 10% higher than the amount in the food product or foodstuff without addition of such food additive comprising a composition comprising 2 or more plant sRNA extracts of the invention, more preferably the amount is at least 20%, 50%, 100% or 200% higher than in the food product or foodstuff without addition of such food additive comprising a composition comprising 2 or more plant sRNA extracts of the invention. In a yet further preferred embodiment, such food products or foodstuffs contain amounts of 2 or more plant sRNA extracts which are each at least 10% higher than the amounts in the food product or foodstuff without addition of such food additive comprising a composition comprising 2 or more plant sRNA extracts of the invention, more preferably the amounts are each at least 20%, 50%, 100% or 200% higher than in the food product or foodstuff without addition of such food additive comprising a composition comprising 2 or more plant sRNA extracts of the invention.

In a further preferred embodiment, such food products or foodstuffs contain an amount of total plant miRNA which is at least 10% higher than the amount in the food product or foodstuff without addition of such food additive containing one or more specific miRNAs, more preferably the amount is at least 20%, 50%, 100% or 200% higher than in the food product or foodstuff without addition of such containing one or more specific miRNAs.

In a yet further preferred embodiment, such food products or foodstuffs contain an amount of a specific plant miRNA which is at least 10% higher than the amount of the specific plant miRNA in the food product or foodstuff without addition of such food additive containing one or more specific miRNAs, more preferably the amount is at least 20%, 50%, 100% or 200% higher than in the food product or foodstuff without addition of such containing one or more specific miRNAs.

Animal feed including pet food compositions advantageously include food intended to supply necessary dietary requirements, as well as treats (e.g., dog biscuits) or other food supplements. The animal feed comprising the composition according to the invention may be in the form of a dry composition (for example, kibble), semi-moist composition, wet composition, or any mixture thereof. Alternatively or additionally, the animal feed is a supplement, such as a gravy, drinking water, yoghurt, powder, suspension, chew, treat (e.g., biscuits) or any other delivery form.

Food compositions are preferably administered orally. Dietary supplements are preferably administered orally.

Therefore, in a yet further embodiment, the present invention relates to a food product comprising a plant sRNA extract, a composition comprising 2 or more plant sRNA extracts or a plant miRNA as defined above.

Therefore, in a yet further embodiment, the present invention relates to a dietary supplement comprising a plant sRNA extract, a composition comprising 2 or more plant sRNA extracts or a plant miRNA as defined above.

In a yet further embodiment, the present invention relates to a method for producing a second food product, comprising adding to a first food product a plant sRNA extract, a composition comprising 2 or more plant sRNA extracts or a plant miRNA as defined herein.

For example, a plant sRNA extract or a composition comprising 2 or more plant sRNA extracts as defined above may be added to a beverage and may be mixed, resulting in a beverage containing or enriched with plant sRNA extract(s). In case the first food product does not contain plant sRNA, the second food product contains plant sRNA(s).

Therefore, the first food product is in a preferred embodiment a food product which does not contain plant sRNA and/or plant miRNA and/or plant sRNA extract(s).

In another preferred embodiment, the first food product contains plant sRNA and/or plant miRNA. In one preferred embodiment, such second food product contain an amount of plant sRNA which is at least 10% higher than the amount in the first food product, more preferably the amount is at least 20%, 50%, 100% or 200% higher than in first food product.

In a further preferred embodiment, such second food product contains an amount of total plant miRNA which is at least 10% higher than the amount in the first food product, more preferably the amount is at least 20%, 50%, 100% or 200% higher than in the first food product.

In a yet further preferred embodiment, such second food product contains an amount of a specific plant miRNA which is at least 10% higher than the amount in the first food product, more preferably the amount is at least 20%, 50%, 100% or 200% higher than in the first food product.

Dietary supplements may be delivered in any suitable format. In preferred embodiments, dietary supplements are formulated for oral delivery. The ingredients of the dietary supplement of this invention are contained in acceptable excipients and/or carriers for oral consumption. The actual form of the carrier, and thus, the dietary supplement itself, is not critical. The carrier may be a liquid, gel, gelcap, capsule, powder, solid tablet (coated or non-coated), tea, or the like.

In other embodiments, the dietary supplement is provided as a powder or liquid suitable for adding by the consumer to a food or beverage. For example, in some embodiments, the dietary supplement can be administered to an individual in the form of a powder, for instance to be used by mixing into a beverage, or by stirring into a semi-solid food such as a pudding, topping, sauce, puree, cooked cereal, or salad dressing, for instance, or by otherwise adding to a food e.g. enclosed in caps of food or beverage container for release immediately before consumption.

The dosage of plant miRNA, plant sRNA extracts for use according to the invention or composition comprising 2 or more plant sRNA extracts as food additive, of course, vary depending upon known factors, such as its mode and route of administration; the age, health and weight of the recipient; the nature and extent of the symptoms; the kind of concurrent treatment; the frequency of treatment; and the effect desired which can be determined by the expert in the field with normal trials, or with the usual considerations regarding the formulation of a miRNA or a plant sRNA extract or a composition comprising 2 or more plant sRNA extracts. Typically, dosage amounts of the plant miRNA or plant sRNA extract or composition comprising 2 or more plant sRNA extracts may vary from 0,1 mg/kg body weight to 500 mg/kg body weight, depending upon the route of administration. Preferably, the dosage amount is 1 to 500, 10 to 500, 10-450, 10-400, 10-300, 10-200, 10-100, 50-400, 50-400, 50-350, 50-300, 50-200, 100-400, 100-300 or 100-200 mg/kg body weight. Typically, the indicated dosage is the dosage per day.

The dosage of plant miRNA or plant sRNA extract for use according to the invention or a composition comprising 2 or more plant sRNA extracts in a pharmaceutical composition can be determined by the expert in the field with normal preclinical and clinical trials, or with the usual considerations regarding the formulation of pharmaceutical composition. Typically, dosage amounts of the plant miRNA or plant sRNA extract or composition comprising 2 or more plant sRNA extracts may vary from 0,1 mg/kg body weight to 500 mg/kg body weight, depending upon the route of administration. Preferably, the dosage amount is 1 to 500, 10 to 500, 10-450, 10-400, 10-300, 10-200, 10-100, 50-400, 50-400, 50-350, 50-300, 50-200, 100-400, 100-300 or 100-200 mg/kg body weight. Typically, the indicated dosage is the dosage per day.

The pharmaceutical compositions may be administered as single dose or multiple doses.

The compositions according to the present invention may be in any galenic form that is suitable for administering to the animal body including the human body, more in particular in any form that is conventional for oral administration, e.g. in solid form, for example tablets, pills, granules, dragees, capsules, and effervescent formulations such as powders and tablets, or in liquid form, for instance in the form of solutions, emulsions or suspensions, for example as pastes and oily suspensions. The pastes may be filled into hard or soft shell capsules, whereby the capsules feature e.g. a matrix of (fish, swine, poultry, cow) gelatin, plant proteins or lignin sulfonate. Examples for other application forms are forms for transdermal, parenteral, topical or injectable administration. The pharmaceutical compositions may be in the form of controlled (delayed) release formulations.

For example, a DOTAP formulation of a composition comprising 3 plant sRNA extracts for intravenous injection was used successfully in the Examples.

In one preferred embodiment the compositions according to the invention are in the form of a formulation for intravenous injection.

In a further preferred embodiment the compositions according to the invention are in the form of a tablet, a pill, a granule, a dragee, a capsule or an effervescent formulation.

In a yet further preferred embodiment, the present invention relates to a method of treating or preventing an inflammatory disease, comprising administering to a patient in need thereof a pharmaceutically effective amount of plant sRNA extract, a composition comprising 2 or more plant sRNA extracts, or plant miRNA.

In a yet further preferred embodiment, the present invention relates to a method of preventing an inflammatory disease, comprising administering to a human a plant sRNA extract, a composition comprising 2 or more plant sRNA extracts, or plant miRNA, which is preferably comprised in a dietary supplement, food or food additive. The human is in one preferred embodiment a patient in need thereof. In another embodiment the human is a healthy person, in particular a person not suffering from an inflammatory disease.

The dietary supplement may be administered as single dose or multiple doses.

In a yet further embodiment of the food product of the present invention or the method of method for producing a second food product, the miRNA is *F. vesca* miR168.

### Figures Legend

- Figure 1:: Examples of the sRNA profiles obtained according to Example 1. A. Ladder profile showing the six RNA markers in nucleotides (nt); B. Profile obtained by running a total RNA sample encompassing the high molecular weight fraction, identifiable by the 28S and 18S peaks, and the sRNA fraction of low molecular weight, on the left of the electropherogram. C-G. Examples of the sRNA fractions used in the experiments here presented. H. Electropherogram of a sample that has been excluded after analysis since high molecular weight RNA contamination (18S and 28S) is present.

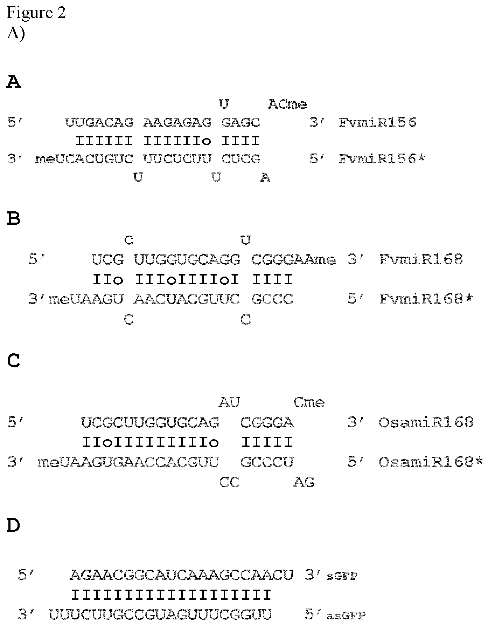

- Figure 2:: A: shows the sequences of duplex structures of *Fragaria vesca* FvmiR156 (A) FvmiR168 (B), *Oryza sativa* OsamiR168 (C) and a sGFP (D). me: methyl group. B: shows Examples of sRNA profiles' size determination obtained according to Example 1.
- Figure 3:: A: Effects of different plant sRNA extracts on T cell proliferation. MLR results are shown. Any treatment is statistically significant with respect to the control (DC + T w/o sRNA treatment): B: Effects of different plant sRNA extracts on T cell proliferation on a mouse cell line. MLR results are shown. Proliferation is presented as stimulation index, Percentage Stimulation above background is determined for each stimulated sample, through comparison with results from an unstimulated sample.

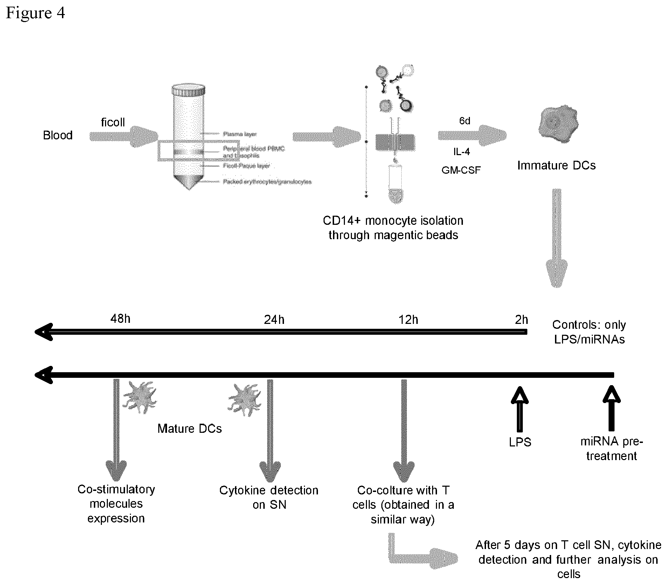

- Figure 4:: shows the Experimental design to obtain dendritic cells for miRNA treatment and to evaluate the effect of plant miRNA on stimulated DC.

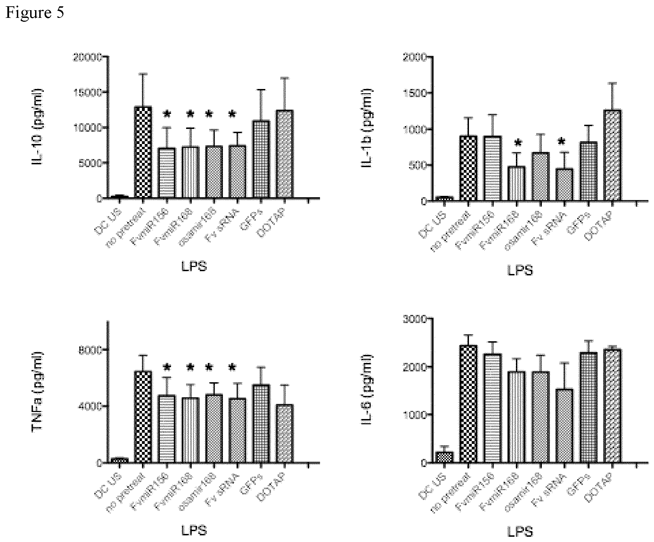

- Figure 5:: Effects of plant miRNA treatment in DC immune function. Effects of miRNA treatment on IL-1β, TNFα, IL-10 and IL-6 production. Mean + SD, N=5, *p<0.05, **p<0.01 T test, $p<0.05, Kruskal Wallis test, treatment vs no pretreatment

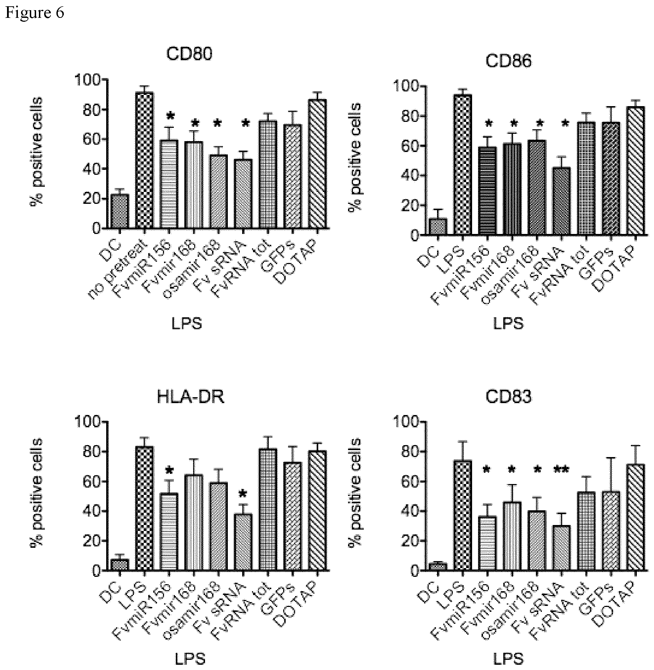

- Figure 6:: Effects of plant miRNA treatment in DC immune function. Effects of miRNA treatment on costimulatory molecules expression. Mean + SD, N=3, *p<0.05, **p<0.01 T test, $p<0.05, Kruskal Wallis test, treatment vs no pretreatment

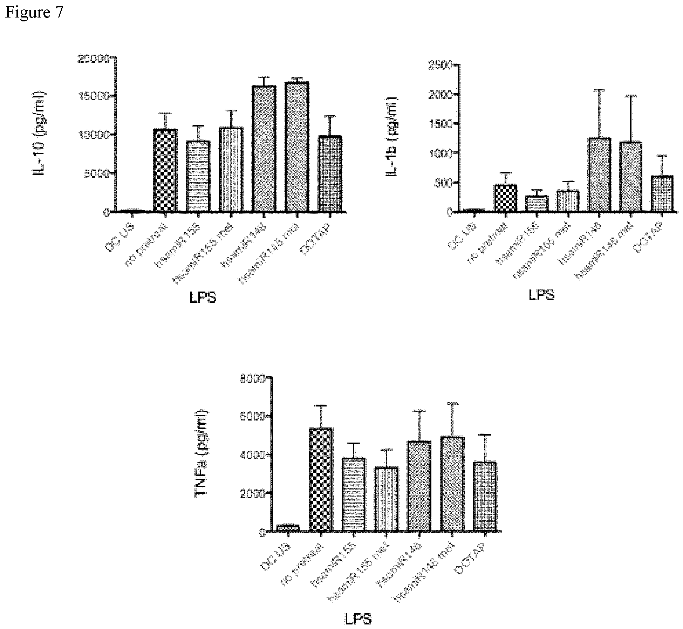

- Figure 7:: Effects of endogenous human miRNA treatment in DC immune function. Effects miRNA treatment on IL-1β, TNFα, IL-10 production. Mean + SD, N=5, *p<0.05, **p<0.01 T test, $p<0.05, Kruskal Wallis test, treatment vs no pretreatment

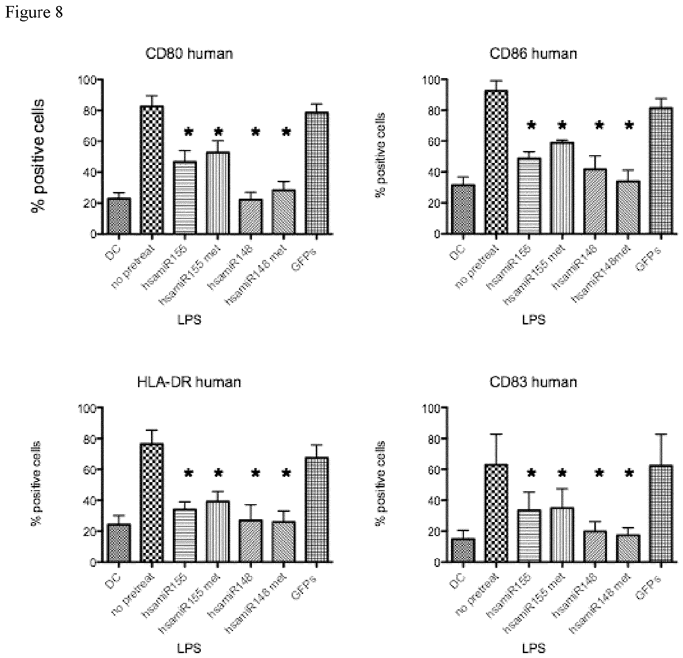

- Figure 8:: Effects of endogenous human miRNA treatment in DC immune function. Effects of miRNA treatment on costimulatory molecules expression. Mean + SD, N=5, *p<0.05, **p<0.01 T test, $p<0.05, Kruskal Wallis test, treatment vs no pretreatment

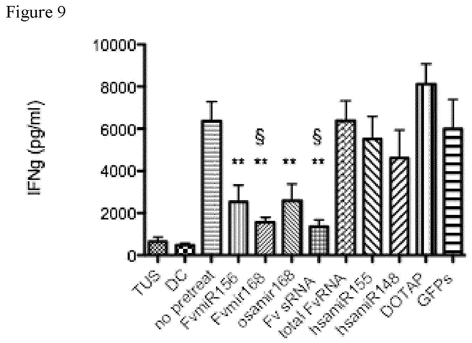

- Figure 9:: Ability of T cells to produce INFγ in response to LPS-loaded DCs previously exposed to different plant and human miRNAs as well as total FvsRNA fraction. Mean + SD, N=5, *p<0.05, **p<0.01 T test, §p<0.05, Kruskal Wallis test, treatment vs no pretreatment.

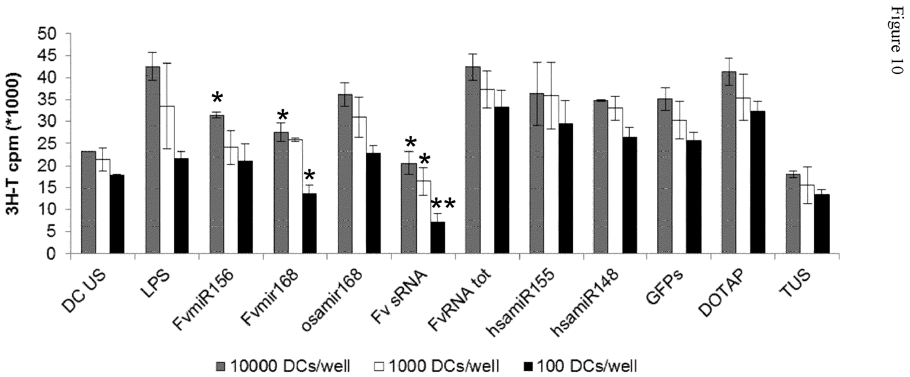

- Figure 10:: Effects of miRNA treatment on T cell proliferation. MLR results after treatment with different plant and human miRNA as well as total FvRNA fraction are shown. Mean + SD, N=4, *p<0.05, **p<0.01 T test, treatment *vs* not pretreatment MLR assay showed that FvmiR168 and FvsRNA modulate T cell proliferation.

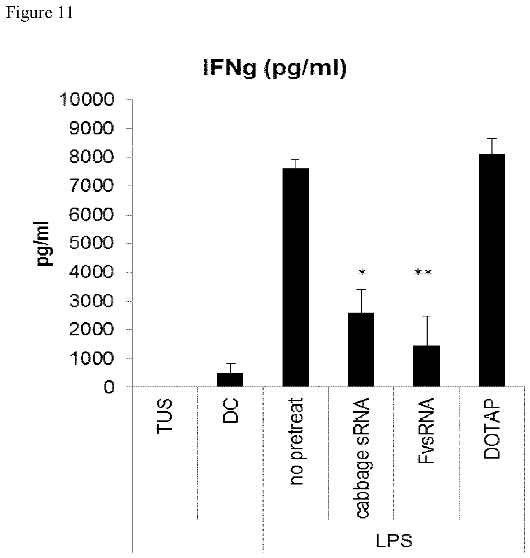

- Figure 11:: Ability of T cells to produce INF-γ in response to LPS-loaded DCs previously exposed to total sRNA extract. Mean + SD, N=5, *p<0.05, **p<0.01 T test, treatment vs not pretreatment

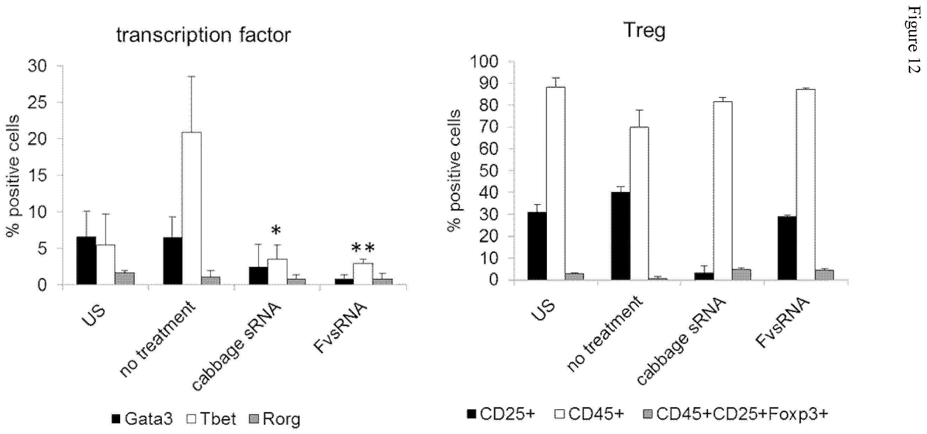

- Figure 12:: T cell sub-population characterization. PBMCs were treated with LPS in presence or absence of 10ng/ml *F. vesca* and cabbage leaves sRNA extracts. After 5 days CD4⁺ cells were analyzed by flow cytometry for presence of Th1 (Tbet), Th2 (Gata3), Th17 (Rorγ) or Treg (CD45⁺CD25^{high}Foxp3⁺) cells. Data are presented as mean±sd (N=3). *p<0.05, **p<0.01 T test, treatment vs not pretreatment.
- Figure 13:: Effects of sRNA fractions on T cell proliferation. MLR results after treatment with different plant sRNA fractions as well as total sRNA samples are shown. Proliferation is presented as stimulation index, Percentage Stimulation above background is determined for each stimulated sample, through comparison with results from an unstimulated sample. Mean + SD, N=6, straight bars: p<0.05, T test, treatment vs not pretreatment.

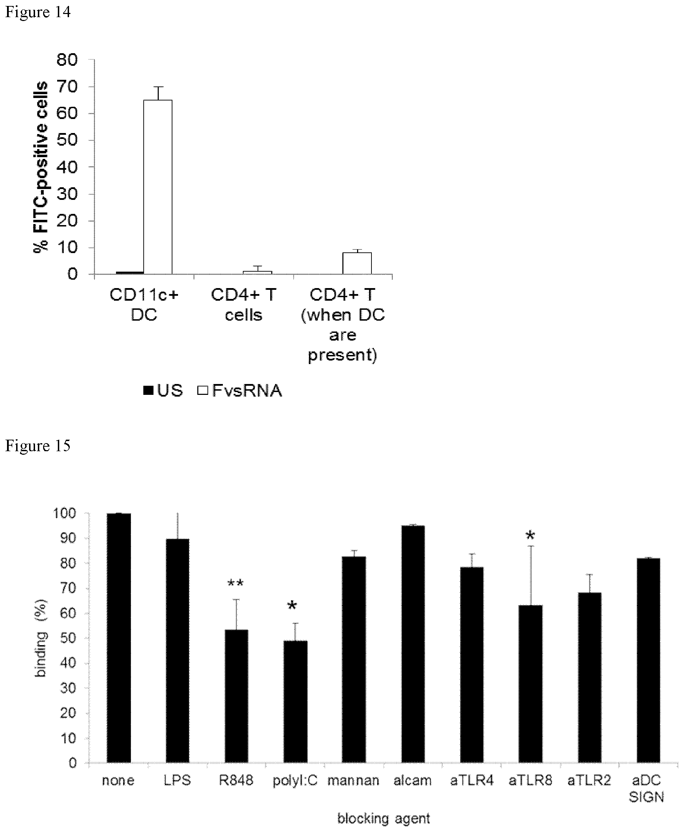

- Figure 14:: Binding of sRNA to immune cells. Cells were co-cultured for 2 hours with green-labeled sRNA (10 ng/ml) and then analyzed by flow cytometry. Data are presented as mean±sd (N=3).
- Figure 15:: Blocking experiment. Cells were pre-treated for 1h with the different blocking agents and then exposed for 2 hours to the fluorescein-labeled F. *vesca* sRNA. Cells were then washed and analyzed for positivity to FITC by flow cytometry. Data are presented as percentage of ligand where binding of FITC-nucleic acids without blocking is set as 100% of binding mean±sd (N=3). *p-value <0.05, T-test, blocked vs not-blocked cells (none) .
- Figure 16:: Effects of human epithelial sRNA extract on T cell proliferation. MLR results after treatment with different plant sRNA fractions as well as total sRNA samples are shown. Proliferation is presented as stimulation index, Percentage Stimulation above background is determined for each stimulated sample, through comparison with results from an unstimulated sample. Mean + SD, N=4, ** p<0.01, T test, treatment vs not pretreatment.
- Figure 17:: Blocking experiment. DCs were pre-treated for 1h with different blocking agents (PolyI:C (TLR3 ligand), ssRNA40 (TLR8 ligand), FvsRNA, pool sRNA, human epithelial sRNA extract) and then exposed for 2 hours to the fluorescein-labeled sRNA or TLR ligands. Cells were then washed and analyzed for positivity to FITC by flow cytometry. Data are presented as percentage of binding where binding of FITC-nucleic acids without blocking is set as 100% of binding. Mean±sd (N=7), *p-value <0.05, T-test, blocked vs not-blocked cells (none) .

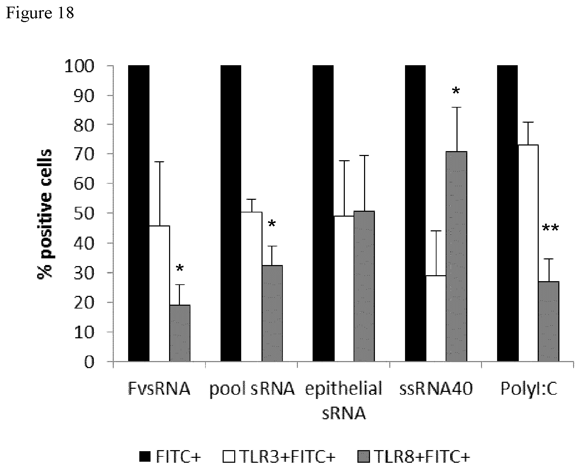

- Figure 18:: Co-localization experiment. Cells were treated for 1h with the fluorescein-labeled sRNA or PolyI:C or ssRNA40. Cells were then washed, labeled with TLR3 antibody and analyzed for double positivity to FITC and APC (TLR3) by flow cytometry of FITC and PE (TLR8). Data are presented as percentage of positive cells where total FITC cells are set as 100%. Mean±sd (N=6), *p-value <0.05, **p-value<0.01, T-test, TLR3+ vs TLR8+ cells.
- Figure 19:: Blocking experiment. DCs were pre-treated for 1h with PolyI:C (TLR3 ligand), or ssRNA40 (TLR8 ligand), and then exposed for 2 hours to the fluorescein-labeled sRNA fractions. Cells were then washed and analyzed for positivity to FITC by flow cytometry. Data are presented as percentage of binding where binding of FITC-nucleic acids without blocking is set as 100% of binding. Mean±sd (N=6), straight bars, p-value <0.01, grey bars p-value <0.05, T-test, blocked vs not-blocked cells (none).
- Figure 20:: Gene Expression analysis using real-time PCR to assess TRIF (TICAM1) (A) and IFNβ (B) expression. Monocyte derived DCs have been exposed for 1h to the different sRNA and then stimulated with LPS. Gene expression has been measured on total RNA extracted after 4h of stimulation.
- Figure 21:: Effect of plant extract in EAE. Pool sRNA significantly ameliorated the clinical signs and disease progression of EAE. Analysis was performed by one way ANOVA test indicating that curves are statistically different (*p<0,05). In particular clinical score is statistically significant in pool sRNA vs untreated mice and in pool sRNA vs vehicle treated mice at day 8 and 15 after immunization. The results are shown as the means ± SD of two independent experiments.
- Figure 22:: Effect of treatment with plant extract on inflammation and demyelination in the spinal cord of EAE mice. Upper photographs) H&E staining displayed a decrease of the number of infiltrates (arrows) in the white matter in the spinal cord of pool sRNA-treated mice compared to untreated or vehicle treated mice. Lower photographs) LFB staining indicated a similar pattern after pool-sRNA treatment (arrows).

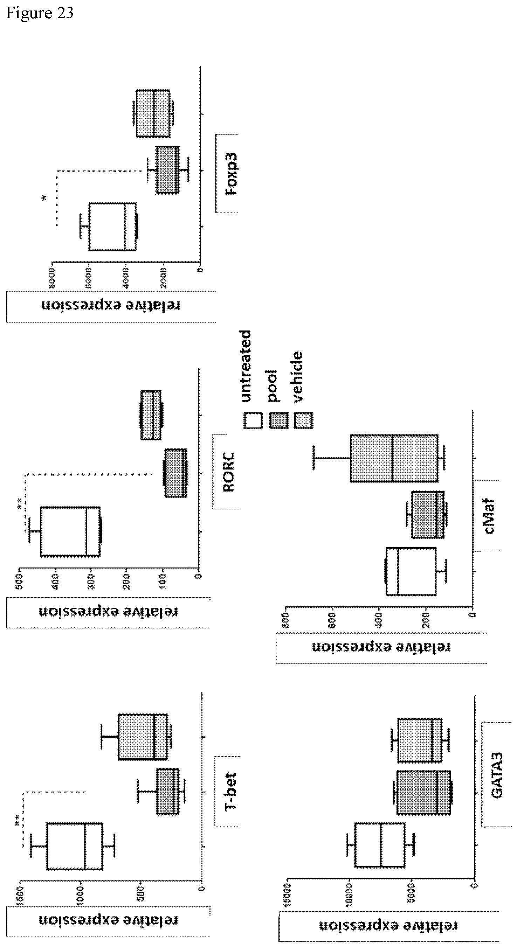

- Figure 23:: Characterization of EAE cellular population in terms of T cell population Th1 (Tbet), Th2 (GATA3), Th17 (RORc), Treg (FoxP3) and cMAF factor which directly regulates the production of IL-10.

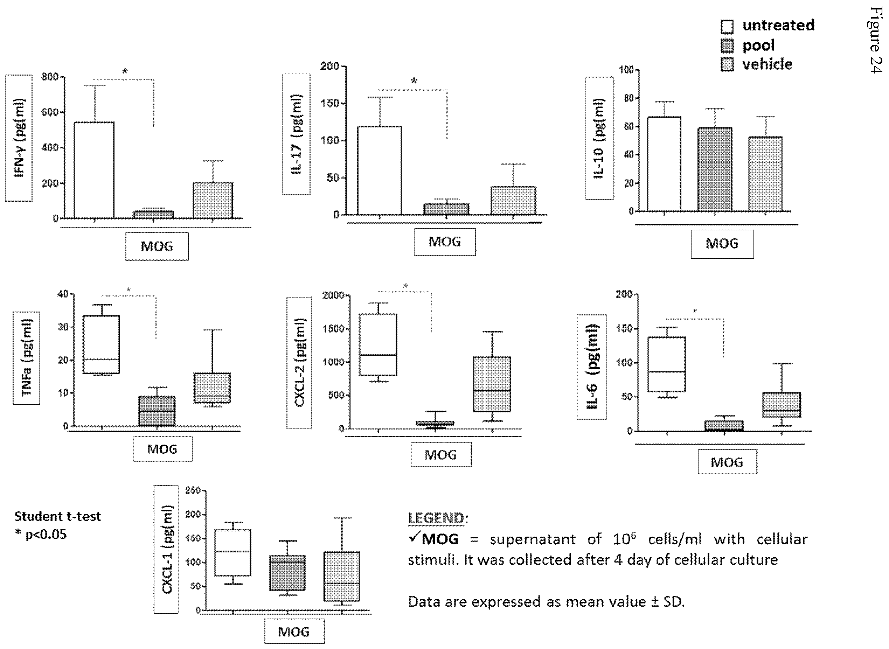

- Figure 24:: Characterization of cytokine expression on EAE lymph nodes homogenates.

### Sequences

| | |
|---|---|
| SEQ ID No. 1 | corresponds to the sequence of methylated FvmiR156 |
| | (5'-UUGACAGAAGAGAGUGAGCACmeth-3'). |
| SEQ ID No. 2 | corresponds to the sequence of methylated FvmiR156* |
| | (5'-AGCUCUUUCUCUUUCUGUCACUmeth-3'). |
| SEQ ID No. 3 | corresponds to the sequence of methylated FvmiR168. |
| | (5'-UCGCUUGGUGCAGGUCGGGAAmeth-3') |
| SEQ ID No. 4 | corresponds to the sequence of methylated FvmiR168* |
| | (5'-CCCGCCUUGCAUCAACUGAAUmeth-3'). |
| SEQ ID No. 5 | corresponds to the sequence of methylated osamiR168. |
| | (5'-UCGCUUGGUGCAGAUCGGGACmeth-3'). |
| SEQ ID No. 6 | corresponds to the sequence of methylated osamiR168* |
| | (5'-GAUCCCGCCUUGCACCAAGUGAAUmeth-3'). |
| SEQ ID No. 7 | corresponds to the sequence of small duplex sGFP. |
| | (5'-AGAACGGCAUCAAAGCCAACU-3' (sGFP)). |
| SEQ ID No. 8 | corresponds to the sequence of the second strand of small duplex |
| | sGFP (3'-UUGGCUUUGAUGCCGUUCUUUU-5' (asGFP)). |

### Examples

### Example 1: Preparation of a plant sRNA extracts and synthesis of miRNAs miR156, miR168, osamiR168 and small duplex sGFP (Figures 1 and 2)

Plant sRNAs were extracted using the mirPremier microRNA Isolation Kit (Sigma-Aldrich) according to the manufacturer's protocol. We collected several plant materials, encompassing the plant kingdom: strawberry fruits from *Fragaria vesca* (cv. Hawaii 4), blueberry, raspberry fruits, apple skin, cabbage leaves and flowers; Traminer grape; fern leaves; *Arabidopsis thaliana* leaves; wild rice and corn. All of them were pulverized in liquid nitrogen. One hundred milligrams were mixed with the buffer provided in the kit. The small RNA fraction was separated from the high molecular weight RNA by the use of columns included in the kit. Small RNA was finally eluted using RNAse-free water.

After each extraction, samples will be analyzed using the Agilent RNA 6000 Nano Kit through the BioAnalyzer instrument. Examples of the sRNA profiles used in the patent are showed in Figure 1. Figure 1A shows the six RNA markers in nucleotides (nt); Figure 1B shows the profile obtained by running a total RNA samples encompassing the high molecular weight fraction, identifiable by the 28S and 18S peaks, and the sRNA fraction of low molecular weight, on the left of the electropherogram. Figure 1C-1G panels show examples of the sRNA fractions used in the experiments here presented. Figure 1H shows a sample that has been excluded after the analysis since high molecular weight RNA contamination (18S and 28S) is present. Furthermore we analyzed each sRNA preparation using the Agilent Small RNA kit. Through BioAnalyzer analysis we characterized the size of the sRNA present in each fraction we use in the next experiments. Size determination has been assessed on the basis of the retention time with respect to the known sizes and timing of the ladder peaks. The fraction of interest is between 10 and 120 nucleotides (nt) for all the plant sRNA extracts (Figure 2 B).

Synthetic plant small RNA duplexes (Figure 2 A) were synthesized by Sigma Genosys and purified by HPLC. The annealing of small RNA strands and the methylation at 3' was performed as described by Sigma oligo synthesis service (http://www.sigmaaldrich.com/life-science/custom-oligos/sirna-oligos.html). Upon arrival the small RNA duplexes were resuspended in RNAse-free water to the appropriate concentration.

### Example 2: Evaluation of sRNAs effects on the T cell proliferation (Figure 3).

In a Mixed Lymphocyte Reaction (MLR) assay, human CD4+ T cells were co-cultured with allogenic human monocyte-derived DCs in presence or not (positive control) of the different plant sRNA extracts. The MLR is a functional assay which measures the proliferative response of lymphocytes from one individual (the responder) to lymphocytes from another individual (the stimulator). In detail, peripheral blood mononucleated cells were collected by Ficoll gradient centrifugation from healthy donor buffy coat. CD14⁺ monocytes were then isolated by a positive magnetic selection and differentiate in immature DCs in medium containing IL-4 and GM-CSF. After 5 days, such monocyte-derived DCs were collected and used for the following challenges experiments. For MLR, DCs were co-cultured in the presence of CD4+ T cells with or without the presence of sRNA extracts. In each experiment (this or the following) sRNAs or miRNAs are complexed with DOTAP, a liposomal transfection agent, which facilitates nucleic acid entry into the cells (Carvalho et al., TLR3 essentially promotes protective class I-restricted memory CD8+ T-cell responses to Aspergillus fumigatus in hematopoietic transplanted patients. Blood. 2012 Jan 26;119(4):967-77; Bourquin C et al., Immunostimulatory RNA oligonucleotides induce an effective antitumoral NK cell response through the TLR7. J Immunol. 2009 Nov 15;183(10):6078-86). DOTAP is also added to the control cells to exclude cross reactivity).

Any plant sRNA extracts tested, used at the more physiological concentration retrieved in host biofluids (10 ng/ml,) were able to attenuate the T cell proliferation (Figure 3A).

Moreover, we tested the same plant sRNA extracts for their ability to limit T cell proliferation in mice using a mouse cell line. The plant sRNA extracts tested were able to limit T cell proliferation, wherein sRNA extracts from cabbage leaves, fern, apple skin were the most effective (Figure 3B).

### Example 3: Evaluation of miRNAs effects on the immune cells properties and their ability to respond to an inflammatory stimulus

In *in vitro* experiments, human monocyte-derived DCs were exposed to the purified miRNAs (or *Fragaria vesca* pure sRNA extracts) and then pre-treated DCs were challenged with purified LPS, the lipopolysaccharide layer of bacterial wall. This experiment allowed determining the sRNA effect on the activation process and maturation of the DC through co-stimulatory molecules analysis by flow cytometry and detection of soluble factors such as cytokines by DC on culture medium. Co-stimulatory molecules expressions on DC cell surface are markers of proper activation and maturation of the immune cell. Increasing levels of these molecules promote DC-T cells contact and antigen presentation thus activating T cells and their survival, priming the proper adaptive immune response. Cytokines could enhance the cellular response (i.e pro-inflammatory cytokines as IL-6, TNFα, IL-1β, IL-12p70, IL-23, INFγ, IL-17), promote type-2 response and thus favoring antibody production (IL-4, IL-13, IL-5) or down-regulate inflammatory process (IL-10, TGFβ).

In particular, comparing the levels of CD80, CD86, CD83 and the class II immuno-histocompatibility complex (MHCII o HLA-DR) of DC pre-exposed to miRNAs or treated with LPS alone evidence a possible attenuation effects of the specific miRNAs. In parallel by evaluating the released cytokine profiles the ability of DCs to properly respond to the stimuli was assessed. Measuring the level of pro-inflammatory (IL-6, TNFα, IL-1β, IL-12p70) and anti-inflammatory (IL-10) cytokines, allowed appreciation of the immuno-modulatory ability of the specific miRNAs. For all treated DC, 24h- IL-1β, TNFα, IL-10 production and 48h- CD80, CD86, CD83 and MHCII expression were analyzed. The cells were then used in mixed lymphocyte reaction, where treated DCs were co-cultured in the presence of CD4+ T cells. INEγ production was evaluated on culture supernatants after 5 days.

The general experimental design is shown in Figure 4.

In a first set of experiments dendritic cells (DC) were treated with the following agents, respectively:
- *Fragaria vesca* specific small duplex miR156 (FvmiR156) (SEQ ID Nos. 1 and 2; Figure 2A)
- *Fragaria vesca* small duplex miR168 (FvmiR168) (SEQ ID Nos. 3 and 4; Figure 2B)
- rice small duplex osamiR168 (SEQ ID Nos. 5 and 6; Figure 2C)
- *Fragaria vesca* total sRNAs fraction (FvsRNA)
- small duplex chemically modified green fluorescent protein sequence (sGFP; SEQ ID Nos. 7 and 8; Figure 2D) (these already served as negative control for many groups (see Robbins M, 2008, Hum Gen Ther));
- *F. vesca* total high molecular weight RNA fraction (FvRNA)
- *E. coli* LPS (positive control)
- *E. coli* LPS after 2 h pre-exposure to the all nucleic acids above

Several miRNAs concentrations were tested, ranging from the physiological (10ng/ml) to the common experimental concentrations for nucleic acids (10µg/ml, Liu X1 et al. (2010)). MicroRNA-148/152 impair innate response and antigen presentation of TLR-triggered dendritic cells by targeting CaMKIIα. J Immunol. 15;185(12):7244-51; Carvalho et al. TLR3 essentially promotes protective class I-restricted memory CD8+ T-cell responses to Aspergillus fumigatus in hematopoietic transplanted patients. Blood. 2012 Jan 26;119(4):967-77; Bourquin C et al. Immunostimulatory RNA oligonucleotides induce an effective antitumoral NK cell response through the TLR7. J Immunol. 2009 Nov 15;183(10):6078-86). Even if the strongest inhibitory effects were seen at the highest concentrations, in order to avoid possible saturation effects of the system the Example showed results using the more physiological concentration of miRNAs retrieved in host biofluids (Figure 5 and 6). While the high molecular weight RNA fraction does not affect DCs activation by LPS, plant miRNAs and plant sRNA fraction modulate both cytokine and co-stimulatory expression.

In particular, plant sRNAs significantly attenuate IL-1β, TNFα and IL-10 production by LPS, as shown in Figure 5. Moreover, FvmiR68 showed the strongest effects.

In addition, it could be shown that plant sRNAs limit the increase of CD80, CD86 and CD83 expression (Figure 6).

In a further set of experiments (Figure 7 and 8), in order to investigate the possible effect of the presence of the methyl group in the plant miRNA sequence, which is not present in endogenous DC human miRNAs, two human miRNAs (miR148, miR155 and its star form miR155*), known to affect DC response to LPS (for a review, Zhan and Wu, Functional regulation of monocyte-derived dendritic cells by microRNAs. Protein Cell. 2012 Jul;3(7):497-507) were used. DCs were treated with the following agents, respectively:
- human miR155
- human miR155 methylated
- human miR148
- human miR148 methylated

The methylated miRNAs were synthetized by adding a methyl group at the 2'OH group of the 3' terminus.

The methyl group did not change the ability of human miRNAs to modulate the DC response to LPS.

In further experiments to evaluate if not only sRNA fractions but also synthetic plant miRNA could modulate T cell proliferation, prior LPS treatment DCs were exposed for 2 h to:
- *Fragaria vesca* small duplex miR168 (FvmiR168) (SEQ ID Nos. 3 and 4; Figure 2B)
- rice small duplex osamiR168 (SEQ ID Nos. 5 and 6; Figure 2C)
- *Fragaria vesca* total sRNAs fraction (FvsRNA)
- hsamiR155
- hsamiR148
and then co-cultured with CD4⁺ T cells. sRNA- and miRNA-treated-DCs seem to have a lower immunostimulatory ability since LPS-induced IFN-γ is less produced by T cells (Figure 9). MLR assay showed that FvmiR168 and FvsRNA modulate T cell proliferation (Figure 10).

### Example 4: Evaluation of sRNA effects on the immune cells properties and their ability to respond to an inflammatory stimulus (Figures 4; 11-12)

To corroborate the effects shown for plant sRNA extracts human monocyte-derived DCs were exposed to the *Fragaria vesca* and cabbage leaves sRNA extracts and then pre-treated DCs were challenged with purified LPS and then exposed to T cells to evaluate the modulation of IPNγ production induced by LPS. Plant sRNA treated-DCs seem to have a lower immunostimulatory ability since LPS-induced IFN-y is less produced by T cells (Figure 11). We also evaluated the T cells subpopulation present after treatment. By flow cytometry we revealed the T cell population by measuring the expression of their specific transcription factors. While the reduction of Tbet confirmed the diminishing in Th1 response to LPS, a slightly increased of Treg (Foxp3 expressing cells) is shown (Figure 12).

### Example 5: Evaluation of plant sRNA fractions on T cell proliferation: sRNA with size <60 or 70 nucleotides decreases T cell proliferation in response to an inflammatory stimulus (Figure 13)

Using a 12% polyacrylamide gel electrophoresis (PAGE), we fractionated the plant sRNA samples. In each gel run, size determination has been assessed using two different size markers: the Low Range sRNA ladder and the microRNA ladder (both from New Englands BioLabs). Band purification and extraction were performed using the MEGAclear kit from Life Technologies. Size and purity of each sRNA fraction were checked using the Agilent Small RNA kit.

As before, sRNA fractions are complexed with DOTAP, a liposomal transfection agent, which facilitates nucleic acid entry into the cells. With those preparation, in a Mixed Lymphocyte Reaction (MLR) assay, human CD4⁺ T cells were co-cultured with allogenic human monocyte-derived DCs stimulated with LPS in presence or not (positive control) of the different plant sRNA fractions (10 ng/ml).

In Figure 13, we expressed the ability of T cells to proliferate as stimulation index (proliferation in respect to background). sRNA fractions ranging 10-60 or 15-60 nucleotides negatively modulate T cells proliferation in response to LPS.

### Example 6: Evaluation of plant sRNA binding on the immune cells (Figure 14-19)

In this experiment we evaluated the ability of plant sRNAs to bind DCs and T cells used in the previous examples. Thus we fluorescein-labeled the plant sRNA samples using the 5'End-Tag kit (Vendor Laboratories) following the manufacturer's instruction. Firstly, we exposed purified DCs and T cells or a co-culture of both to FvsRNA and we analyzed the percentage of positive green cells by flow cytometry. Plant sRNA contact the dendritic cells but not T cells (Figure 14).

We then performed a blocking experiments to evaluate if a specific receptor was involved in such binding. we specifically blocked receptors for several DC antigens by using the specific ligands or an antibody against the receptor.

In particular we used 10 µg/ml of:
- LPS (Sigma-Aldrich), antigen for TLR4
- Resiquimod (R848) (InvivoGen) the synthetic analog to RNA, antigen for TLR7/8
- Low molecular weight (LMW) Polyinosinic:polycytidylic acid (polyI:C) (InvivoGen), the synthetic analog of dsRNA (viral), antigen for TLR3.
- Mannan from *S. cerevisiae* (Sigma-Aldrich)
- Blocking antibody against TLR4
- Blocking antibody against TLR8
- Blocking antibody against DC-SIGN
- Blocking antibody against ALCAM/CD166 antigen, such a negative control. It is expressed on activated T cells, activated monocytes, epithelial cells, fibroblasts, neurons, melanoma cells, and also in sweat and sebaceous glands but not involved in immune response.

DCs were pre-treated for 1h with the different blocking agents and then exposed for 2 hours to the fluorescein-labeled *F*. *vesca* sRNA. Cells were then washed and analyzed for positivity to FITC by flow cytometry.

PolyI:C (TLR3, viral dsRNA) and R848 (TLR8) partially block the binding (Figure 15).

Since the first experiments with *F*. *vesca* sRNA indicated a possible role of TLR3 and partially of TLR8 we extended the test on all the active sRNA samples, and using as non-plant sRNA, sRNA extracted from Caco-2 cell lines. We firstly tested human epithelial sRNA in their ability or not to diminish T cell proliferation driven by LPS as plant sRNA did (see Figure 3). As shown in the figure, human epithelial sRNA extract are not able to limit T cell proliferation as plant sRNAs (Figure 16).

Recently, experiments focused on tumor environment-derived miRNA binding to immune cells used as TLR7/8 ligand the synthetic single strand small RNA (19 nu) ssRNA40 (see Muller Fabbri, et al. (2012) PNAS, 109(31): E2110-E2116). Thus, we introduced ssRNA40 instead of R848 as receptor agonist. Before starting, to check our experiment setting we tested the binding of ssRNA40 to TLR8 by blocking the receptor using an antibody against TLR8 (not shown).

In the example shown, DCs were pre-treated for 1h with different blocking agents
- PolyI:C (TLR3 ligand),
- ssRNA40 (TLR8 ligand),
- strawberry FvsRNA,
- a pool of sRNA comprising 1/3 sRNA extract from cabbage leaves, 1/3 sRNA extract from fern leaves, and 1/3 sRNA extract from apple skin,
- human epithelial sRNA as control.
and then exposed for 2 hours to the fluorescein-labeled sRNA (the above mentioned) or TLR ligands. Cells were then washed and analyzed for positivity to FITC by flow cytometry. Data are presented as percentage of binding where binding of FITC-nucleic acids without blocking is set as 100% of binding.

Contact of plant sRNA are inhibited by both PolyI:C and ssRNA40, indicating that TLR3 and TLR8 are both involved in binding (Figure 17).

We corroborated our results by performing a co-localization experiment. After treatment with fluorescein-labeled sRNA for 2 hours, we intracellularly labeled DCs for TLR3 and TLR8 expression using the FIX and PERM kit (Life Technologies) and APC conjugated anti-TLR3 and PE conjugated anti-TLR8 antibodies. Cells were then analyzed for double positivity to FITC and APC (TLR3) or FITC and PE (TLR8). Data are presented as percentage of positive cells where total FITC cells are set as 100%.

Both plant and non-plant sRNA seems to bind to TLR3 and TLR8 but contact of plant sRNA seems to be mediated mainly by TLR3 and only in part by TLR8 (Figure 18).

To evaluate which fractions of the plant sRNA extract samples bind to the DCs, we used the sRNA fractions purified in the Example 4 in a blocking experiment as described above. DCs were pre-treated for 1h with PolyI:C (TLR3 ligand), or ssRNA40 (TLR8 ligand), and then exposed for 2 hours to the fluorescein-labeled sRNA fractions (in the examples, apple skin and FvsRNA fractions are shown, Figure 19). Cells were then washed and analyzed for positivity to FITC by flow cytometry. PolyI:C block the binding of 10-60 nu sRNA but not that of higher size sRNA.

### Example 7: Gene Expression analysis to assess TRIF (TICAM1) and IFNβ expression downstream to TLR3 signal (Figure 20)

Monocyte-derived DCs have been exposed for 1h to the different sRNA and then stimulated with LPS. Gene expression has been measured on total RNA extracted after 4h of stimulation with LPS. Data have been expressed as fold increase. Pretreatment with small RNA induces down-regulation of both the adaptor protein TRIF-encoding gene and IFNβ transcription (Figure 20).

### Example 8: Exogenous plant extracts ameliorate experimental autoimmune encephalomyelitis (Figures 21-24)

We studied plant sRNA involvement on the pathogenesis of experimental autoimmune encephalomyelitis (EAE) induced in C57BL/6 mice by immunization with myelin oligodendrocyte glycoprotein peptide 35-55 (MOG peptide), a model of multiple sclerosis (E. Mix et al. 2010, Neurobiol.;92: 386-404). In particular we investigated the effect of intravenous injection of plant extracts in EAE pathology, by evaluating the neurological clinical scores, assaying T cells proliferation, DCs phenotype as well as histopathological scores.

sRNA isolated from cabbage leaves, fern and apple skin (i.e. plant sRNA extracts from cabbage leaves, fern and apple skin) were combined together as indicated before and then, were dissolved in phosphate buffered saline and DOTAP Liposomal Transfection Reagent (vehicle) to give the final dose of 10 µg/ml in 150 µl per mouse. sRNA pool or vehicle were given by intravenous injections at the early stage of the disease on day 3 post immunization (dpi) and every 4 days for the entire duration of the disease (up to 25 days after immunization). Two independent experiments were conducted. 12 mice were used for each experiment and they were randomly assigned to 3 different experimental groups:
- mice treated with pool sRNA
- mice treated with vehicle
- mice untreated.

The effect of plant extracts on severity of EAE was daily examined. There was no significant difference in body weight between three groups during the experimental period. Mice from both groups developed the disease with the typical clinical signs.

However, as showed in Figure 21 pool sRNA treatment significantly attenuated the clinical manifestation of EAE when compared to untreated and vehicle-treated mice. The mean of disease onset (day 8 p.i.) and maximum (day 15 p.i.) EAE score from the pool sRNA group was significantly lower compared to control groups (p<0,05). These results indicated that plant sRNA extract administration conferred protection against EAE, promoting a less severe disease.

EAE is mediated by the infiltration of autoreactive immune cells into the central nervous system (CNS) that results in inflammation and demyelination of motoneurons with consequent paralysis. Following EAE induction, both untreated and sRNA or vehicle-treated mice had distinct areas of immune cell infiltration in the spinal cord as revealed by hematoxylin and eosin (H&E) staining (Figure 22, upper photographs). However, sRNA-treated mice had visually less infiltrates than untreated or vehicle-treated mice. The degree of myelin loss was assessed by Luxol Fast Blue (LFB) cresyl violet staining that revealed more numerous and larger plaques of demyelination at the site of inflammatory cell infiltrates in the spinal cord of untreated and vehicle-treated mice than in pool sRNA-treated ones (figure 22, lower photographs).

These results indicate that the less severe disease developed by plant extract-treated mice is associated with improvement histological outcomes in the spinal cord. In conclusion our results showed that plant sRNA extracts attenuate the pathology development of EAE induced by MOG immunization. This improvement was evident through several measures of EAE pathology, including: a decreased neurological deficit score; reduced inflammatory cell infiltration and demyelination in the spinal cord. Moreover, characterization of cellular population in terms of T cell population Th1 (Tbet), Th2 (GATA3), Th17 (RORc), Treg (FoxP3) and cMAF factor which directly regulates the production of IL-10. The analysis shows that "pool" plant sRNA extract treatment inhibit the differentiation of Th1 and Th17, the cells responsible for EAE (Figure 23, 24) and a lack of increase of IL-10, corroborated by the invariate level of cMAF.

The following embodiments are disclosed:
1. A plant sRNA extract for use as an immunosuppressive agent.
2. The plant sRNA extract for use of item 1, wherein the sRNA has a length of 200 nucleotides or less, preferably a length of 120 nucleotides or less, preferably a length between 10 and 120 nucleotides.
3. The plant sRNA extract for use of item 1 or 2, wherein the sRNA has a length of between 10 and 70, 10 and 60, 15 and 60, 20 and 60, or between 10 and 50 nucleotides.
4. The plant sRNA extract for use of any of items 1, 2 or 3, wherein the sRNA is miRNA.
5. The plant sRNA extract for use of any of items 1 to 4, wherein the plant sRNA extract is selected from an apple sRNA extract, a blueberry sRNA extract, a raspberry sRNA extract, a cabbage sRNA extract, an *Arabidopsis thaliana* sRNA extract, a grape sRNA extract, a rice sRNA extract, in particular a wild rice sRNA extract, a corn sRNA extract, a pine sRNA extract, a fern sRNA extract and a sage sRNA extract.
6. The plant sRNA extract for use of any of items 1 to 5, wherein the plant sRNA extract is selected from an apple sRNA extract, cabbage sRNA extract, strawberry sRNA extract and fern sRNA extract,
   preferably wherein
   the apple sRNA extract is an sRNA extract from apple skin, or
   the cabbage sRNA extract is an sRNA extract from cabbage leaves or cabbage flower, more preferred from cabbage leaves, or
   the fern sRNA extract is an sRNA extract from fern leaves, or
   the strawberry sRNA extract is an sRNA extract from strawberry fruit.
7. A composition comprising 2 or more plant sRNA extracts, preferably wherein the composition comprises 2 or more plant sRNA extracts selected from an apple sRNA extract, a blueberry sRNA extract, a raspberry sRNA extract, a cabbage sRNA extract, an *Arabidopsis thaliana* sRNA extract, a grape sRNA extract, a rice sRNA extract, in particular a wild rice sRNA extract, a corn sRNA extract, a pine sRNA extract, a fern sRNA extract and a sage sRNA extract,
   more preferably
   wherein the composition comprises, preferably consists of two or more sRNA extracts selected from strawberry sRNA extract, namely *Fragaria vesca* sRNA extract, cabbage sRNA extract, fern sRNA extract and apple sRNA extract.
8. A composition according to item 7, which comprises, preferably consists of, cabbage leaves sRNA extract, fern leaves sRNA extract and apple skin sRNA extract, and optionally strawberry fruit sRNA extract.
9. The plant sRNA extract for use according to any of items 1 to 6, or the composition of item 8, wherein the sRNA comprises methylated sRNA, more preferably sRNA or miRNA methylated at the 2'OH group of the 3' terminus.
10. The composition according to item 8 or 9, for use as an immunosuppressive agent.
11. A plant sRNA extract, preferably as defined in any of items 2 to 6 and 9, or a composition according to item 7, 8 or 9, for use in a method for treating and/or preventing an inflammatory disease,
   in particular wherein the inflammatory disease is selected from the group consisting of chronic prostatitis, Glomerulonephritis, Hypersensitivities, Pelvic inflammatory disease, Reperfusion injury, Sarcoidosis, Vasculitis, Interstitial cystitis, normocomplementemic urticarial vasculitis, pericarditis, myositis, anti-synthetase syndrome, scleritis, macrophage activation syndrome, Bechet's Syndrome, PAPA Syndrome, Blau's Syndrome, gout, adult and juvenile Still's inflammatory disease, cryropyrinopathy, Muckle -Wells syndrome, familial cold-induced auto-inflammatory syndrome, neonatal onset multisystemic inflammatory disease, familial Mediterranean fever, chronic infantile neurologic, cutaneous and articular syndrome, systemic juvenile idiopathic arthritis, Hyper IgD syndrome, Schnitzler's syndrome, TNF receptor-associated periodic syndrome (TRAPSP), gingivitis, periodontitis, hepatitis, cirrhosis, pancreatitis, myocarditis, vasculitis, gastritis, gout, gouty arthritis, and inflammatory skin disorders, selected from the group consisting of psoriasis, atopic dermatitis, eczema, rosacea, urticaria, and acne.
12. The plant sRNA extract or composition for use according to item 11, wherein said inflammatory disease is an autoimmune disease.
13. The plant sRNA extract or composition for use according to item 12, wherein said autoimmune disease is selected from the group consisting of multiple sclerosis, rheumatoid arthritis; psoriatic arthritis, discoid lupus erythematosus, systemic lupus erythematosus (SLE); ulcerative colitis; Crohn's disease; benign lymphocytic angiitis, autoimmune lymphoproliferative syndrome, sarcoidosis, autoimmune thrombocytopenic purpura, idiopathic thrombocytopenic purpura, pure red cell aplasia, Sjogren's syndrome, rheumatic disease, polymyalgia rheumatica, mixed connective tissue disease, inflammatory rheumatism, degenerative rheumatism, extra-articular rheumatism, juvenile arthritis, juvenile rheumatoid arthritis, systemic juvenile idiopathic arthritis, arthritis uratica, muscular rheumatism, chronic polyarthritis, reactive arthritis, Reiter's syndrome, rheumatic fever, relapsing polychondritis, Raynaud's phenomenon, vasculitis, cryoglobulinemic vasculitis, ANCA-associated vasculitis, temporal arteritis, giant cell arteritis, Takayasu arteritis, Behcet's disease, antiphospholipid syndrome, myasthenia gravis, autoimmune haemolytic anaemia, Guillain-Barre syndrome, chronic immune polyneuropathy, chronic inflammatory demyelinating polyneuropathy, autoimmune thyroiditis, insulin dependent diabetes mellitus, type I diabetes, Addison's disease, membranous glomerulonephropathy, polyglandular autoimmune syndromes, Goodpasture's disease, autoimmune gastritis, autoimmune atrophic gastritis, pernicious anaemia, pemphigus, pemphigus vulgaris, cirrhosis, primary biliary cirrhosis, idiopathic pulmonary fibrosis, myositis, dermatomyositis, juvenile dermatomyositis, polymyositis, fibromyositis, myogelosis, celiac disease, celiac sprue dermatitis, immunoglobulin A nephropathy, Henoch-Schonlein purpura, Evans syndrome, atopic dermatitis, psoriasis, psoriasis vulgaris, psoriasis arthropathica, Graves' disease, Graves' ophthalmopathy, scleroderma, systemic scleroderma, progressive systemic scleroderma, diffuse scleroderma, localized scleroderma, Crest syndrome, asthma, allergic asthma, allergy, primary biliary cirrhosis, Hashimoto's thyroiditis, fibromyalgia, chronic fatigue and immune dysfunction syndrome (CFIDS), primary myxedema, sympathetic ophthalmia, autoimmune inner ear disease, autoimmune uveitis, autoimmune chronic active hepatitis, collagen diseases, ankylosing spondylitis, periarthritis humeroscapularis, panarteritis nodosa, polyarteritis nodosa, chondrocalcinosis, Wegener's granulomatosis, microscopic polyangiitis, chronic urticaria, bullous skin disorders, pemphigoid, bullous pemphigoid, cicatricial pemphigoid, vitiligo, atopic eczema, eczema, chronic urticaria, autoimmune urticaria, normocomplementemic urticarial vasculitis, hypocomplementemic urticarial vasculitis, alopecia areata, alopecia universalis, alopecia totalis, Devic's disease, pernicious anemia, childhood autoimmune hemolytic anemia, idiopathic autoimmune hemolytic anemia, refractory or chronic Autoimmune Cytopenias, Prevention of development of Autoimmune Anti-Factor VIII Antibodies in Acquired Hemophilia A, Cold agglutinin disease, Neuromyelitis Optica, Stiff Person Syndrome, gingivitis, periodontitis, pancreatitis, myocarditis, gastritis, gout, gouty arthritis, idiopathic pericarditis, anti- synthetase syndrome, scleritis, macrophage activation syndrome, PAPA Syndrome, Blau's Syndrome, adult and juvenile Still's disease, cryopyrin associated periodic syndrome, Muckle -Wells syndrome, familial cold auto- inflammatory syndrome, neonatal onset multisystem inflammatory disease, chronic infantile neurologic cutaneous and articular syndrome, familial Mediterranean fever, Hyper IgD syndrome, Schnitzler's syndrome, autoimmune retinopathy, age-related macular degeneration, and TNF receptor-associated periodic syndrome (TRAPS),
   preferably wherein said autoimmune disease is multiple sclerosis.
14. The plant sRNA extract or composition for use according to item 11, wherein said inflammatory disease is an allergic disease.
15. The plant sRNA extract or composition for use according to item 14, wherein said allergic disease is selected from the group consisting of allergic respiratory disease, such as bronchial asthma, pediatric asthma, allergic asthma, atopic asthma, aspirin asthma, or allergic bronchitis, allergic nasal disease, such as allergic rhinitis, vernal catarrh, hay fever, or chronic allergic rhinitis, an allergic skin disease, such as atopic dermatitis, an allergic ocular disease, such as hay fever, seasonal allergic conjunctivitis, or chronic allergic conjunctivitis, hypersensitivity pneumonitis, contact dermatitis, and food allergy.
16. The plant sRNA extract or composition for use according to item 11, wherein said inflammatory disease is psoriasis.
17. The plant sRNA extract or composition for use according to any of items 11 to 16, wherein the inflammatory disease is characterized by an increase in expression or production of at least one of inflammatory biomarker, in particular selected from IL-1β and TNFα and/or wherein the inflammatory disease is characterized by a dysregulation of the cytokines or co-receptors in at least one cell and/or tissue of a patient, in particular wherein the at least one cell is a dendritic cell.
18. The plant sRNA extract for use according to any of items 1 to 6, 9 or 11 to 17, wherein said sRNA extract is derived from strawberry, or the composition of any of items 7 to 9, or the composition for use of any of items 11 to 17, wherein said composition comprises a strawberry sRNA extract.
19. The plant sRNA extract for use according to any of items 1 to 6, 9 or 11 to 18 or the composition for use of any of items 11 to 18, wherein said plant sRNA extract or composition is administered in combination with at least one other anti-inflammatory agent.
20. The plant sRNA extract or composition for use according to item 19, wherein said other anti-inflammatory agent is selected from the group consisting of a corticosteroid, a statin, interferon beta, a nonsteroidal anti-inflammatory drug (NSAID), methotrexate, Cyclosporin A and a disease-modifying anti-rheumatic drug (DMARD).
21. The plant sRNA extract for use according to any of items 1 to 6, 9 or 11 to 20 or the composition for use of any of items 11 to 20, or the composition of any of items 7 to 9, wherein said plant sRNA extract or composition is comprised in a food additive or dietary supplement.
22. The plant sRNA extract for use according to any of items 1 to 6, 9 or 11 to 19 or the composition for use of any of items 11 to 19, wherein said plant sRNA extract is comprised in a pharmaceutical composition.
23. A pharmaceutical composition, food additive or dietary supplement, comprising a composition according to any of items 7 to 9, or a plant sRNA extract as defined in any of items 1 to 6 and 9,
   preferably
   wherein the pharmaceutical composition, food additive or dietary supplement comprises a) lipids, more preferably at least one liposome, or b) an exosome.
24. A food product comprising a plant sRNA extract, preferably a plant sRNA extract as defined in any of items 1 to 6 and 9, or a composition according to any of items 7 to 9.
25. A method for producing a second food product, comprising adding to a first food product a plant sRNA extract, preferably a plant sRNA extract as defined in any of items 1 to 6 and 9, or a composition according to any of items 7 to 9.
26. A plant sRNA extract, preferably as defined in any of items 2 to 6 and 9, or composition comprising 2 or more plant sRNA extracts according to any of items 7 to 9, for use as a medicament.

## Claims

1. A dietary supplement comprising: a plant sRNA extract, a composition comprising 2 or more plant sRNA extracts or a plant miRNA,
wherein said plant sRNA extract, composition comprising 2 or more plant sRNA extracts, said plant miRNA exhibit an anti-inflammatory effect.

2. The dietary supplement according to claim 1 wherein the plant sRNA comprises methylated sRNA at the 2'OH group at the 3' terminus and the plant miRNA is methylated at the 2'OH group at the 3' terminus.

3. The dietary supplement according to claim 1 or 2, comprising miR156, miR168, osamiR168 or mixtures thereof.

4. The dietary supplement according to claim 3 wherein the miRNA is miR168, preferably Fragaria vesca (F. vesca) miR168.

5. The dietary supplement according to any one of claims 1-4 wherein dietary supplement comprises a) lipids, more preferably at least one liposome, or b) an exosome.

6. A dietary supplement for use in a method for treating and/or preventing an inflammatory disease, said supplement comprising: a plant sRNA extract, a composition comprising 2 or more plant sRNA extracts or a plant miRNA.

7. A method for producing a second food product, comprising adding to a first food product a plant sRNA extract or a composition comprising 2 or more plant sRNA extracts of a given plant variety for use as an immunosuppressive agent, wherein:
- a plant sRNA extract is the purified small RNA fraction of a plant which has a length of 200 nucleotides or less, optionally dissolved in a suitable solvent,
- the amount of sRNA in said plant sRNA extract in comparison to other RNA is at least 200% higher than in naturally occurring plant material of the same plant variety, and
- the plant sRNA extract comprises sRNA methylated at the 2'OH group of the 3' terminus.
preferably wherein the sRNA of the plant sRNA extract contains less than 10% ribosomal RNA of the same plant variety.

8. The method for producing a second food product of claim 7, wherein the plant sRNA extract comprises the sRNA of a plant which has a length of between 10 and 120 nucleotides.

9. The method for producing a second food product of any of claims 7 to 8, wherein
- the plant sRNA is extracted from leaves, flowers, roots, fruits, seeds or parts thereof, and/or
- the plant is a land plant (*Embriophytae*)*,* in particular a flowering plant, a fern, a seed plant or a pine, and/or the plant is a monocot or dicot.

10. The method for producing a second food product of any of claims 7 to 9, wherein the method comprises adding a plant sRNA extract or a composition comprising 2 or more plant sRNA extract as defined in any of claims 7-9 to a beverage, and optionally mixing, thereby obtaining a beverage containing or enriched with plant sRNA extract(s).

11. The method for producing a second food product of anyone of claims 7 to 10, wherein the first food product does not contain plant sRNA, and the second food product contains plant sRNA.

12. The method for producing a second food product of anyone of claims 7 to 11, wherein the first and second food products:
- are selected from food or feed suitable for consumption by humans or animals, preferably wherein the feed suitable for consumption by animals is selected from extruded and pelleted animal feed, coarse mixed feed and a pet food composition, or is a dry composition, semi-moist composition, wet composition, or any mixture thereof, and/or is a supplement, drinking water, yoghurt, powder, suspension, chew, treat, or biscuit, or
- are selected from beverages, preferably selected from non-alcoholic and alcoholic drinks, liquid preparation to be added to drinking water and liquid food, more preferably wherein the non-alcoholic drinks are selected from soft drinks, sport drinks, fruit juices, lemonades, teas, near-water drinks, milk, other dairy drinks, and diet drinks or
- are solid or semi-solid foods, preferably selected from baked goods, more preferably elected from cakes and cookies, puddings, dairy products, confections, snack foods, frozen confections, novelties, prepared frozen meals, candy, snack products, liquid food, soups, spreads, sauces, salad dressings, prepared meat products, cheese, yoghurt, fat or oil containing foods, and food ingredients.

13. The method for producing a second food product of anyone of claims 7 to 12 wherein the second food product comprises miR156, miR168, osamiR168 or mixtures thereof.

14. The method for producing a second food product of claim 13 wherein the miRNA is miR168, preferably Fragaria vesca (F. vesca) miR168.

15. The second food product obtainable by the method according to any one of claims 6-14.


SEQUENCE LISTING

<110>  Fondazione Edmund Mach

<120>  Plant sRNA extract for use as an immunosuppressive agent

<130>  BE 146974

<150>  PCT/EP2014/058888
<151>  2014-04-30

<160>  8

<170>  PatentIn version 3.5

<210>  1
<211>  21
<212>  RNA
<213>  Artificial Sequence

<220>
<223>  methylated FvmiR156


<220>
<221>  misc_feature
<222>  (21)..(21)
<223>  cm

<400>  1
uugacagaag agagugagca n                                                21


<210>  2
<211>  22
<212>  RNA
<213>  Artificial Sequence

<220>
<223>  methylated FvmiR156*


<220>
<221>  misc_feature
<222>  (22)..(22)
<223>  um

<400>  2
agcucuuucu cuuucuguca cn                                               22


<210>  3
<211>  21
<212>  RNA
<213>  Artificial Sequence

<220>
<223>  methylated FvmiR168


<220>
<221>  misc_feature
<222>  (21)..(21)

<223>  2'-O-methyladenosine

<400>  3
ucgcuuggug caggucggga n                                                21


<210>  4
<211>  21
<212>  RNA
<213>  Artificial Sequence

<220>
<223>  methylated FvmiR168*


<220>
<221>  misc_feature
<222>  (21)..(21)
<223>  um

<400>  4
cccgccuugc aucaacugaa n                                                21


<210>  5
<211>  21
<212>  RNA
<213>  Artificial Sequence

<220>
<223>  methylated osamiR168


<220>
<221>  misc_feature
<222>  (21)..(21)
<223>  cm

<400>  5
ucgcuuggug cagaucggga n                                                21


<210>  6
<211>  24
<212>  RNA
<213>  Artificial Sequence

<220>
<223>  methylated osamiR168*


<220>
<221>  misc_feature
<222>  (24)..(24)
<223>  um

<400>  6
gaucccgccu ugcaccaagu gaan                                             24


<210>  7
<211>  21
<212>  RNA

<213>  Artificial Sequence

<220>
<223>  small duplex sGFP

<400>  7
agaacggcau caaagccaac u                                                21


<210>  8
<211>  21
<212>  RNA
<213>  Artificial Sequence

<220>
<223>  asGFP

<400>  8
uuggcuuuga ugccguucuu u                                                21